# EUROPEAN PATENT SPECIFICATION

(11) **EP 0 808 325 B1**
(45) Date of publication and mention of the grant of the patent: **17.01.2001**
(21) Application number: 95942879.8
(22) Date of filing: 22.11.1995
(51) Int. Cl.: C07J 9/00, C07J 5/00, A61K 31/56

(54) **ANDROSTANE AND PREGNANE SERIES FOR ALLOSTERIC MODULATION OF GABA RECEPTOR**
ANDROSTAN- UND PREGNANSERIEN FÜR DIE ALLOSTERISCHE MODULATION DES GABA-REZEPTORS
SERIES DE L'ANDROSTANE ET DE LA PREGNANE PRODUISANT UNE MODULATION ALLOSTERIQUE DU RECEPTEUR DU GABA

(30) Priority: 23.11.1994 US 346926
(43) Date of publication of application: 26.11.1997
(73) Proprietor: COCENSYS, INC., Irvine, CA 92718 (US)
(72) Inventor: LAN, Nancy, C., South Pasadena, CA 91030 (US); GEE, Kelvin, W., Irvine, CA 92715 (US); BOLGER, Michael, B., Los Alamitos, CA 90720 (US); TAHIR, Hasan, New Delhi 110 044 (IN); PURDY, Robert, San Diego, CA 92122 (US); UPASANI, Ravindra, B., Fothill Ranch, CA 92610 (US)
(74) Representative: Chapman, Paul William
(86) International application number: US9515210
(87) International publication number: WO9616076

(56) References cited:
- WO-A-93/03732
- WO-A-93/05786
- WO-A-94/27608
- US-A- 3 953 429
- US-A- 5 120 723
- US-A- 5 232 917
- US-A- 5 371 077
- PHILLIPPS G H: "STRUCTURE-ACTIVITY RELATIONSHIPS IN STEROIDAL ANAESTHETICS" NOL. MECH. GEN. ANAESTH. GLAXO SYMPOSIUM, 1 January 1974, pages 32-47, XP000600765
- PHILLIPPS G H: "STRUCTURE-ACTIVITY RELATIONSHIPS IN STEROIDAL ANAESTHETICS" JOURNAL OF STEROID BIOCHEMISTRY, vol. 6, no. 5, May 1975, pages 607-613, XP000600716
- R. PURDY ET AL: "Synthesis, Metabolism and Pharmacological Activity of 3.alpha.-Hydroxy Steroids Which Potentiate GABA-Receptor Mediated Chloride Ion Uptake in Rat Cerebral Cortical Synaptoneurosomes" JOURNAL OF MEDICINAL CHEMISTRY., vol. 33, no. 6, June 1990, WASHINGTON US, pages 1572-1581, XP002044395

## Description

The present invention is directed to methods, compositions, and compounds for modulating animal brain excitability via the gamma-aminobutyric acid A (GABA_{A}) receptor-chloride ionophore complex (GRC). Specifically, the present invention is directed to methods, compositions, and compounds for modulating brain excitability through binding to the neurosteroid receptor site on the GRC.

### Background of the Invention

Brain excitability is defined as the level of arousal of an animal, a continuum that ranges from coma to convulsions, and is regulated by various neurotransmitters. In general, neurotransmitters arc responsible for regulating the conductance of ions across neuronal membranes. At rest, the neuronal membrane possesses a potential (or membrane voltage) of approximately -80 mV, the cell interior being negative with respect to the cell exterior. The potential (voltage) is the result of ion (K⁺, Na⁺, Cl⁻, organic anions) balance across the neuronal semipermeable membrane. Neurotransmitters are stored in presynaptic vesicles and are released under the influence of neuronal action potentials. When released into the synaptic cleft, an excitatory chemical transmitter such as acetylcholine will cause membrane depolarization (change of potential from -80 mV to -50 mV). This effect is mediated by postsynaptic nicotinic receptors which are stimulated by acetylcholine to increase membrane permeability to Na⁺ ions. The reduced membrane potential stimulates neuronal excitability in the form of a postsynaptic action potential.

In the case of the GRC, the effect on brain excitability is mediated by GABA, a neurotransmitter. GABA has a profound influence on overall brain excitability because up to 40% of the neurons in the brain utilize GABA as a neurotransmitter. GABA regulates the excitability of individual neurons by regulating the conductance of chloride ions across the neuronal membrane. GABA interacts with its recognition site on the GRC to facilitate the flow of chloride ions down an electrochemical gradient of the GRC into the cell. An intracellular increase in the levels of this anion causes hyperpolarization of the transmembrane potential, rendering the neuron less susceptible to excitatory inputs (i.e., reduced neuron excitability). In other words, the higher the chloride ion concentration in the neuron, the lower the brain excitability (the level of arousal).

It is well-documented that the GRC is responsible for the mediation of anxiety, seizure activity, and sedation. Thus, GABA and drugs that act like GABA or facilitate the effects of GABA (e.g., the therapeutically useful barbiturates and benzodiazepines (BZs) such as Valium) produce their therapeutically useful effects by interacting with specific regulatory sites on the GRC.

It has also been observed that a series of steroid metabolites interact with the GRC to alter brain excitability (Majewska, M.D. *et al., Science* 232:1004-1007 (1986); Harrison, N.L. *et al., J. Pharmacol. Exp. Ther. 241* :346-353 (1987)). Prior to the present invention, the therapeutic usefulness of these steroid metabolites was not recognized by workers in the field due to an incomplete understanding of the potency and site of action. Applicants' invention relates in part to a pharmaceutical application of the knowledge gained from a more developed understanding of the potency and site of action of certain steroid compounds.

The ovarian hormone progesterone and its metabolites have been demonstrated to have profound effects on brain excitability (Backstrom, T. *et al., Acta Obstet. Gynecol. Scand*. *Suppl.* 130:19-24 (1985); Pfaff, D.W. and McEwen, B.S., *Science* 219:808-814 (1983); Gyerrnek *et al., J*. *Med*. *Chem. 11*:117 (1968); and Lambert, J. *et al., Trends Pharmacol.* 8:224-227 (1987)). The levels of progesterone and its metabolites vary with the phases of the menstrual cycle. It has been well documented that progesterone and its metabolites decrease prior to the onset of menses. The monthly recurrence of certain physical symptoms prior to the onset of menses has also been well documented. These symptoms, which have become associated with premenstrual syndrome (PMS) include stress, anxiety, and migraine headaches (Dalton, K., *Premenstrual Syndrome and Progesterone Therapy,* 2nd edition, Chicago: Chicago yearbook, 1984). Patients with PMS have a monthly recurrence of symptoms that are present in premenses and absent in postmenses.

In a similar fashion, a reduction in progesterone has also been temporally correlated with an increase in seizure frequency in female epileptics (i.e., catamenial epilepsy; Laidlaw, J., "Catamenial epilepsy," *Lancet,* 1235-1237 (1956)). A more direct correlation has been observed with a reduction in progesterone metabolites (Rosciszewska *et al., J. Neurol. Neurosurg. Psych. 49*:47-51 (1986)). In addition, for patients with primary generalized petit mal epilepsy, the temporal incidence of seizures has been correlated with the incidence of the symptoms of premenstrual syndrome (Backstrom, T. *et al., J. Psychosom. Obstet. Gynaecol. 2*:8-20 (1983)). The steroid deoxycorticosterone has been found to be effective in treating patients with epileptic spells correlated with their menstrual cycles (Aird, R.B. and Gordan, G., *J. Amer. Med. Soc. 145*:715-719 (1951)).

A syndrome also related to low progesterone levels is postnatal depression (PND). Immediately after birth, progesterone levels decrease dramatically leading to the onset of PND. The symptoms of PND range from mild depression to psychosis requiring hospitalization; PND is associated with severe anxiety and irritability. PND-associated depression is not amenable to treatment by classic antidepressants and women experiencing PND show an increased incidence of PMS (Dalton, K., 1984).

Collectively, these observations imply a crucial role for progesterone and deoxycorticosterone and more specifically their metabolites in the homeostatic regulation of brain excitability, which is manifested as an increase in seizure activity or symptoms associated with catamenial epilepsy, PMS, and PND. The correlation between reduced levels of progesterone and the symptoms associated with PMS, PND, and catamenial epilepsy (Backstrom *et al.,* 1983; Dalton, K., 1984) has prompted the use of progesterone in their treatment (Mattson *et al.,* in *Advances in epileptology: XVth Epilepsy International Symposium,* Raven Press, New York, 279-282, 1984, and Dalton, K., 1984). However, progesterone is not consistently effective in the treatment of the aforementioned syndromes. For example, no dose-response relationship exists for progesterone in the treatment of PMS (Maddocks, *et al., Obstet. Gynecol. 154*:573-581 (1986); Dennerstein, *et al., British Medical Journal, 290*:16-17 (1986)).

The publications and references referred to above and hereafter in this specification are incorporated herein by reference.

### Summary of the Invention

According to a first aspect of the present invention, there is provided a compound selected from the group consisting of:
3α-hydroxy-3β-(3'-methyl-but-3'-en-1'-ynyl)-5β-pregnan-20-one;
3α-hydroxy-3β-(3'-methyl-but-3'-en- 1'-ynyl)-5α-pregnan-20-one;
3β-(cyclopropyl)ethynyl-3α-hydroxy-5β-pregnan-20-one; and
3α,21-dihydroxy-3β-fluoromethyl-5α-pregnan-20-one; or
a physiologically acceptable 3-ester, 20-ester, 21-ester, 3,20-diester, or 3,21-diester thereof.

According to a second aspect of the present invention, there is provided a compound selected from the group consisting of:
3α,21-dihydroxy-3β-trifluoromethyl-19-nor-5β-pregnan-20-one, 21-hemisuccinate, sodium salt;
3β,20β-dihydroxy-21-methyl-5α-pregnane, bis hemisuccinate;
3α,21-dihydroxy-3β-ethenyl-5α-pregnan-20-one, 21-hemisuccinate;
3α,21-dihydroxy-3β-trifluoromethyl-5β-pregnan-20-one, 21 hemifumarate sodium salt;
3α,21-dihydroxy-3β-trifluoromethyl-5)-pregnan-20-one, methyl 21-succinate;
3α,21-dihydroxy-3β-trifluoromethyl-5β-pregnan-20-one, 21-propionate;
bis (3α,21-dihydroxy-3β-trifluoromethyl-5β)-pregnan-20-one) 21-hemisuccinate; and
bis (3α,21-dihydroxy-3β-ethynyl-5β-pregnan-20-one) 21-hemisuccinate.

Preferred compounds of the invention include 3α-hydroxy-3β-(3'methyl-but-3'-en-1'-ynyl)-5β-pregnan-20-one; 3α,21-dihydroxy-3β-trifluoromethyl-19-nor-5β-pregnan-20-one, 21-hemisuccinate, sodium salt; and 3β-(cyclopropyl)ethynyl-3α-hydroxy-5β-pregnan-20-one.

In a third aspect, the invention provides a pharmaceutical composition, comprising an effective amount of a compound of the first and second aspects of the invention; and a pharmaceutically acceptable carrier or diluent.

In further aspects, the invention provides the use of a compound of the first and second aspects of the invention in the manufacture of a medicament for
- the treatment of stress or anxiety in an animal subject;
- alleviating seizure activity in an animal subject;
- reducing or alleviating insomnia in an animal subject;
- the treatment of mood disorders, preferably depression, in an animal subject;
- the treatment of pre-menstrual syndrome or post-natal depression in an animal subject; and
- inducing anaesthesia in an animal subject.

The present invention is directed to methods, compositions, and compounds for modulating brain excitability. More particularly, the invention relates to the use of 3α-hydroxylated steroid derivatives, acting at a newly identified site on the GR complex, to modulate brain excitability in a manner that will alleviate stress, anxiety, insomnia, mood disorders (such as depression) that are amenable to GR-active agents, and seizure activity. Compositions and compounds effective for such treatment are within the scope of the invention.

The compounds used in and forming part of the invention are modulators of the excitability of the central nervous system as mediated by their ability to regulate chloride ion channels associated with the GABA receptor complex. Applicants' experiments have established that the compounds used in and of the invention have anticonvulsant and anxiolytic activity similar to the actions of known anxiolytic agents such as the BZs, but act at a distinct site on the GR complex.

The relationship of endogenous metabolites of progesterone to processes associated with reproduction (estrus cycle and pregnancy) is well established (Marker, R.E., Kamm, O., and McGrew, R-V., "Isolation of epi-pregnanol-3-one-20 from human pregnancy urine," *J. Am. Chem. Soc. 59*:616-618 (1937)). Prior to the present invention, however, it was not recognized how to treat disorders by modulating brain excitability through the use of progesterone metabolites. Therefore, this invention is directed to methods, compositions, and compounds to treat disorders by modulating brain excitability using the compounds of this invention. Representative disorders treated in the present invention are epilepsy, anxiety, pre-menstrual syndrome (PMS), post-natal depression (PND), mood disorders (such as depression) that are amenable to GR-active agents, and insomnia. The compounds of the invention can also be used to induce anesthesia.

### Detailed Description of the Preferred Embodiments

The compounds of and used in the invention are derivatives of various 3α-hydroxylated-pregnan-20-ones; 3α-21-pregnanediol-20-ones; 3α-20-pregnanediols; and 3α-hydroxylated-androstanes, and ester derivatives thereof, which derivatives are referred to as prodrugs. The expression "prodrug" denotes a derivative of a known direct acting drug, which derivative has enhanced delivery characteristics and therapeutic value as compared to the drug, and is transformed into the active drug by an enzymatic or chemical process; see Notari, R.E., "Theory and Practice of Prodrug Kinetics," *Methods in Enzymology, 112*:309-323 (1985); Bodor, N., "Novel Approaches in Prodrug Design," *Drugs of the Future, 6*(3):165-182 (1981); and Bundgaard, H., "Design of Prodrugs: Bioreversible-Derivatives for Various Functional Groups and Chemical Entities," in *Design of Prodrugs* (H. Bundgaard, ed.), Elsevier, New York (1985). It should be noted that some of the synthetic derivatives forming part of the present invention may not be true prodrugs because, in addition to the above characteristics, they also possess intrinsic activity. However, for purposes of this application they will be referred to as prodrugs.

Our studies (Gee, K.W. *et al., European Journal of Pharmacology,* 136:419-423 (1987)) have demonstrated that the 3α-hydroxylated steroids used in the invention are orders of magnitude more potent than others have reported (Majewska, M.D. *et al.* (1986) and Harrison, N.L. *et al.* (1987)) as modulators of the GR complex. Majewska *et al.* and Harrison *et al.* teach that the 3α-hydroxylated-5-reduced steroids are only capable of much lower levels of effectiveness. Our *in vitro* and *in vivo* experimental data demonstrate that the high potency of these steroids allows them to be therapeutically useful in the modulation of brain excitability via the GR complex. The most potent steroids useful in the present invention include derivatives of major metabolites of progesterone and deoxycorticosterone. These steroids can be specifically used to modulate brain excitability in stress, anxiety, insomnia, mood disorders (such as depression) that are amenable to GR-active agents, and seizure disorders in a therapeutically beneficial manner. Furthermore, we have demonstrated that these steroids interact at a unique site on the GR complex which is distinct from other known sites of interaction (i.e., barbiturate, BZ, and GABA) where therapeutically beneficial effects on stress, anxiety, sleep, mood disorders and seizure disorders have been previously elicited (Gee, K.W. and Yamamura, H.I., *in In Central Nervous System Disorders,* pages 123-147, D.C. Horvell, ed., 1985; Lloyd, K.G. and Morselli, P.L., in *Psychopharmacology: The Third Generation of Progress,* pages 183-195, H.Y. Meltzer, ed., Raven Press, N.Y., 1987). These compounds are desirable for their duration, potency and oral activity (along with other forms of administration).

### Definitions

In accordance with the present invention and as used herein, the following terms are defined with the following meaning, unless explicitly stated otherwise.

The term "alkyl" refers to saturated aliphatic groups including straight chain, branched chain, and cyclic groups, all of which may be optionally substituted. Suitable alkyl groups include methyl, ethyl, and the like, and may be optionally substituted.

The term "alkenyl" refers to unsaturated groups which contain at least one carbon-carbon double bond and includes straight chain, branched chain, and cyclic groups, all of which may be optionally substituted.

The term "alkynyl" refers to unsaturated hydrocarbon groups which contain at least one carbon-carbon triple bond and includes straight chain, branched chain, and cyclic groups all of which may contain additional unsaturation. When present at the 3β-position, the alkynyl groups may be substituted with a halogenated or unhalogenated C₁ radical, a C₂-C₆ saturated or unsaturated, halogenated or unhalogenated straight chain radical, a C₃-C₆ cyclic (cycloalkyl) radical, or C₅-C₆ aromatic radical or a 4, 5, or 6-membered C- or N- attached heterocyclic radical containing 1, 2 or 3 heteroatoms selected from the group consisting of oxygen, nitrogen, and sulfur, excluding heterocyclic radicals with two or more adjacent O or S atoms. Preferred alkynyl groups have two to four carbon atoms.

The term "acyl" refers to the alkanoyl group -C(O)R where R is alkyl, alkenyl, alkynyl, aryl, or aralkyl.

The term "optionally substituted" or "substituted" refers to groups substituted by one to three substituents, independently selected from lower alkyl, aryl, alkenyl, alkynyl, alkoxy, amino, thio, halo, haloalkyl, trihaloalkyl, acyl, nitro, hydroxy, and keto.

The term "pharmaceutically acceptable esters" refers to esters of the compounds of the first aspect of the invention derived from the combination of a compound of this invention and an organic or inorganic acid.

Further preferred compounds are compounds of the invention which are esters of hydroxyl groups at positions 3 and/or 20. Preferred esters are those described by their corresponding acids: acetic, propionic, maleic, fumaric, ascorbic, pimelic, succinic, glutaric, bismethylene-salicylic, methanesulfonic, ethane-di-sulfonic, oxalic, tartaric, salicylic, citric, gluconic, itaconic, glycolic, p-aminobenzoic, aspartic, glutamic, gamma-amino-butyric, α-(2-hydroxyethylamino)-propionic, glycine and other α-amino acids, phosphoric, sulfuric, glucuronic, and 1-methyl-1,4-dihydronicotinic.

The following synthetic methods and examples are directed to the preparation of compounds forming part of and used in the present invention.

### Synthetic Methods

The compounds according to the invention may be prepared by any convenient method, e.g. using conventional techniques such as are described in "Steroid Reactions" Djerassi, published in 1963 by Holden-Day, Inc., San Francisco or "Organic Reactions in Steroid Chemistry", Fried and Edwards, published in 1972 by Van Nostrand-Reinhold Co., New York.

The compounds of the invention may be prepared by any suitable technique known in the art.

### General Methods

20-Hydroxy pregnanes were prepared by the reduction of 20-keto pregnanes with conventional reducing agents.

21-Hemisuccinates were prepared from pregnan-20-one derivatives which were first brominated with molecular bromine to obtain the corresponding 21-bromo pregnanes. The bromo compounds were then reacted with various dioic acids, such as succinic acid, in the presence of an amine to yield 21-hydroxy esters. The resulting esters from the dioic acids were then converted to their sodium salts by conventional means.

The esters may be formed using reactions well known in the art between the hydroxyl group on the compounds discussed above with an organic acid, acid halide, acid anhydride, or ester, wherein the organic acids are, for example, acetic, propionic, maleic, fumaric, ascorbic, pimelic, succinic, glutaric, bismethylenesalicylic, methanesulfonic, ethane-di-sulfonic, oxalic, tartaric, salicylic, citric, gluconic, itaconic, glycolic, *p*-aminobenzoic, aspartic, glutamic, γ-amino-butyric, α-(2-hydroxyethylamino)-propionic, glycine and other α-amino acids, phosphoric, sulfuric, glucuronic, and 1-methyl-1,4-dihydronicofinic.

### 3β-Sbstituents

### Halomethyl

The 3β-monohalomethyl compounds of this invention may be prepared by treatment of a 3-spiro-2'oxirane steroid with a source of halide ions in an inert solvent, such as tetramethylammonium halide in toluene, and preferably a proton source, such as acetic acid.

### Saturated or unsaturated alkyl

Other 3-substituted steroids may be prepared by the addition of an organometallic reagent to a 3-ketosteroid in which other reactive functional groups may be protected as necessary. Thus, 3-alkynyl compounds may be prepared by the use of an lithium acetylide in inert solvents or with a reagent prepared in situ from 1,2-dibromoethylene and butyl lithium as the organometallic reagent Likewise, 3-alkenyl compounds may be prepared the reaction of a 3-ketosteroid with a vinyl organometallic reagent such as vinyl magnesium bromide. Compounds in which the unsaturation is removed for the site of reaction such as allyl magnesium bromide may also be used to give compounds containing a 3-alkenyl group. Similarly, the use of an alkyl Grignard such as methyl magnesium iodide will lead to 3-alkyl compounds.

### Trifluoromethyl

The-trifluoromethyl group may be prepared by reaction of a 3-ketosteroid with trimethyltrifluoromethylsilane catalyzed by fluoride ion.

### 21-Oxygenated compounds

### Lead tetraacetate oxidation of 21-methyl

Various compounds of this type may be prepared by a reaction sequence in which a pregnan-20-one is oxidized with lead tetraacetate to give a 21-acetoxy derivative, hydrolysis of the acetate to give a 21-alcohol, and acylation with an appropriate carboxylic acid derivative, such as an anhydride or acid chloride or other reagent capable of replacing the hydrogen of the hydroxyl group, such as methanesulfonyl chloride.

### Pregnan-17-enes

These may be formed by the reaction of a 17-ketosteroid with a Wittig reagent such as the ylide derived from treatment of n-propyltriphenylphosphonium bromide with a strong base such as potassium t-butoxide.

### 3,20-Diols

A pregnan-3,20-diol may be formed by addition of a boron hydride such as diborane to the double bond of a pregnan-17-ene followed by oxidation of the thus formed organoborane with, for example, alkaline hydrogen peroxide, to give the 20-ol. Alternatively, pregnan-3,20-diols may be formed by the reduction of a 20-one group to a 20-ol group. Suitable reagents are hydride reagents such as sodium borohydride or dissolving metals such as sodium in n-propanol and the like.

### Examples (Reference)

### Example 1

### a. 3α-Hydroxy-5β-pregnan-20-one, 20-ketal

A mixture of 3α-hydroxy-5β-pregnan-20-one (10.8 g, 34 mmol), ethylene glycol (45 mL), and triethyl orthoformate (30 mL) was stirred at rt for 5 min. *p*-Toluenesulfonic acid (200 mg) was then added and the stirring was continued at rt for 1.5 hr. The resulting thick paste was poured into a sat. NaHCO₃ solution (250 mL). The precipitated solid was collected by filtration, washed thoroughly with cold water and dried. This semi-dried product was dissolved in CH₂Cl₂ (350 mL) and dried over anhyd. K₂CO₃. The solution of the ketal was then filtered and used as such for the next step.

### b. 5β-Pregnan-3,20-dione, 20-ketal

The above solution of 3α-hydroxy-5β-pregnan-20-one, 20-ketal in CH₂Cl₂ was stirred with N-methylmorpholine-N-oxide (8.8 g, 75 mmol), and powdered 4Å molecular sieves (58 g) under N₂ for 15 min. Tetrapropylammonium perruthenate (400 mg) was then added and the stirring was continued at r.t. for 2 hr. The resulting dark green mixture was passed through a short column of Florisil and eluted with CH₂Cl₂. The fractions containing the product (TLC) were combined and evaporated. The crude product was then crystallized from a mixture of EtOAc:Hx (1:1) to yield the title compound (10.3 g) as long rods.

### c. Preparation of the lithium reagent from 1,2-dibromoethylene

A 100 mL three neck flask equipped with a N₂ gas bubbler, a thermometer, and a dropping funnel was charged with 1,2-dibromoethylene (cis/trans mixture, 98%, Aldrich, 0.164 mL, 2 mmol, mw = 186, d = 2.246). Dry THF (15 mL) was added and the solution was cooled to -78°C in a dry ice-acetone bath. n-BuLi (2.5M in THF, 1.6 mL, 4 mmol) was added dropwise over a period of 10 min. The mixture was stirred at this temperature for 1 hr and the resulting reagent was used immediately for the next step.

### d. 3β-Ethynyl-3α-hydroxy-5β-pregnan-20-one

The above solution of the reagent in THF, which was maintained at -78°C, was treated dropwise with a solution of 5β-pregnan-3,20-dione, 20-ketal (180 mg, 0.5 mmol) in THF (15 mL). The temperature was maintained below - 70°C during the addition. The stirring was continued at this temperature for 15 min (100% conversion as detected by TLC). The cooling bath was removed and the resulting solution was quenched with 2N HCl (pH 6). The solvent was removed and the residue was dissolved in acetone (10 mL). After adding 2N HCl (4 mL) the solution was stirred at ambient temperature for 0.5 h. The mixture was neutralized with dil. NaHCO₃ solution. The precipitated solid (158 mg, 93%) was collected by filtration, washed with water, and dried. The crude product is then purified by crystallization from EtOAc or from a mixture of acetone-hexane to yield the title compound; mp 196-197°C, TLC-R_{f} 0.45 (hexane:acetone 7:3).

### Example 2 (Reference)

### Disodium 3α,20β-dihydroxy-5β-pregnane, bis(hemisuccinate)

A suspension of 3α,20β-dihydroxy-5β-pregnane (Steraloids; 250 mg, 0.78 mmol) in 5 mL of dry pyridine was treated with succinic anhydride (200 mg, 2.0 mmol). The mixture was heated at 100°C for a total of 10 h. An additional 6 mmol of succinic anhydride was added in three portions as the reaction was heated. The dark mixture was concentrated (0.05 mmHg, 30°C) to remove the solvent and then heated at 90°C (0.05 mmHg) to remove excess succinic anhydride. The residue was recrystallized from ether/hexanes giving a solid that consisted mainly of succinic acid. The mother liquor was concentrated and subjected to column chromatography (flash silica gel, eluted with 95/5/0.1 CH₂Cl₂/MeOH/HOAc) giving a white solid which was recrystallized from ether/hexane. The bis(hemisuccinate), mp 81-90°C was carried on to the bis(sodium salt).

The bis(hemisuccinate) (100 mg, 0.192 mmol) was dissolved in a minimum volume of methanol. A solution of NaHCO₃ (2 eq, 33 mg, 0.393 mmol) in 0.6 mL of water was added dropwise. After 3h, the solution was concentrated *in vacuo to* give a white solid.

### Example 3 (Reference)

### 3α-Hydroxy-3β-trifluoromethyl-5α-pregnan-20-one and 3β-Hydroxy-3α-trifluoromethyl-5α-pregnan-20-one

To solution of 5α-pregnan-3,20-dione 20-ethyleneketal (356 mg, 0.987 mmol) in dry THF (5 mL) was added 0.5M F₃CSi(CH₃)₃ (in THF; 2.5 mL, 1.25 mmol). The resulting colorless solution was cooled to O°C, and *n*-Bu₄NF•xH₂O (few crystals) was added. Cooling bath was removed. Evolution of a gas (Me₃SiF) was observed, and the reaction solution turned yellow. The mixture was stirred at room temperature for 30 min. TLC (3:1 hexane/acetone) showed complete consumption of the starting material; the new spot moved almost with solvent-front Subsequently, 1N HCl (~3 mL) was added, and the resulting two-phase mixture was stirred at room temperature overnight. TLC (3:1 hexane/acetone) now displayed a single spot with an R_{f} of ∼0.5. On other hand, when CH₂Cl₂ was used, there were two close spots, the top one being for the minor product. Ether and water were added. The aqueous layer was back-extracted with ether. The combined organics were washed with saturated NaHCO₃ and brine, dried (MgSO₄), filtered, and evaporated under reduced pressure to give a white crystalline (foamy) solid, which was flash-chromatographed employing CH₂Cl₂ as eluant.

Evaporation of early fractions furnished 3α-hydroxy-3β-trifluoromethyl-5α-pregnan-20-one (10 mg).

Further elution of the column gave 3β-hydroxy-3α-trifluoromethyl-5α-pregnan-20-one (200 mg), which on the basis of ¹⁹F NMR and GC-MS also contained 1.5% minor product (i.e., 3α-hydroxy-3β-trifluoromethyl-5α-pregnan-20-one). In order to remove this impurity, recrystallization in hot 60:40 hexane/ethyl acetate was attempted; no crystals were formed. Finally, highly pure 3β-hydroxy-3α-trifluoromethyl-5α-pregnan-20-one (145 mg) could be obtained by subjecting the above 97.5:1.5 mixture to another flash chromatography with CH₂Cl₂, mp 181-3 °C.

### Example 4 (Reference)

### 3β-Hydroxy-3α-ethenyl-5β pregnan-20-one and 3α-Hydroxy-3β-ethenyl-5β-pregnan-20-one

A solution of 5β-pregnan-3,20-dione, 20-ketal (1.18g, 3.3 mmol) in dry THF (20 mL) was treated with vinyl magnesium bromide (1M in THF, 3.7 mmol, 3.7 mL) at -70°. After stirring the mixture at this temp. for 5 min and then at rt for 2.5 h, it was quenched with sat. NH₄Cl solution (10 mL). The solvent was removed and the residue was extracted with EtOAc. The organic layer was, washed with water, diL NaHCO₃ soln., water, and brine. After drying over anhyd. MgSO₄ the solution was filtered and evaporated to yield the crude product (1.2 g). This crude product was then dissolved in acetone (20 mL). After adding IN HCl (10 mL) the solution was stirred at rt for 15 h. The solvents were removed and the residue was extracted with CH₂Cl₂. The organic layer was washed with water, dil. NaHCO₃ soln., water, and brine. After drying over anhyd. MgSO₄ the solution was filtered and evaporated to yield the crude product (890 mg). This crude product was then dissolved in a small amount of CH₂Cl₂ and poured on a column of silica gel. On elution with toluene:acetone mixture (94:6) gave 3α-ethenyl-3β-hydroxy-5β-pregnan-20-one (126 mg) as a first fraction. Further elution with the same solvent mixture yielded 3β-ethenyl-3α-hydroxy-5β-pregnan-20-one (189 mg), m.p. 113-116°.

### Example 5 (Reference)

### a. 3α-Hydroxy-5α-androstan-17-one

To a solution of 3β-hydroxy-5α-androstan-17-one (6g) and diethyl azodicarboxylate (5.04 g) in THF (50 mL) was added trifluoroacetic acid (3.3 g) and the mixture became yellow. Then triphenylphosphine (7.6 g) was added. The reaction turned to colorless and heat was given off. Sodium benzoate was added after 5 min and then water (100 mL) was added. The mixture was extracted with methylene chloride (3x80 mL) and organics was dried over magnesium sulfate. The solvent was removed *in vacuo* and the crude product was hydrolyzed with potassium hydroxide (10%, 10 mL) in methanol (150 mL) for 1 h. Most of the methanol was then removed *in vacuo* and the remaining material was partitioned between methylene chloride and ammonium chloride. The product (4.7 g, 78%) was purified by chromatography (CH₂Cl₂: acetone=9:1).

### b. 3α-Hydroxy-21-methyl-5α-pregn-17(20)(Z)-ene

3α-Hydroxy-5α-androstan-17-one (2 g, 6.9 mmol) was added to Wittig -reagent produced from propyltriphenylphosphium bromide (13.3 g) and potassium t-butoxide (3.9 g) in THF (20 mL). The reaction was refluxed for 12 h and cooled to 25°C. Then a solution of ammonium chloride (60 mL) was added and the organic layer was separated by separatory funnel. The aqueous layer was extracted with methylene chloride (2x50 mL). The organic solution was dried over potassium carbonate and the solvent removed *in vacuo.* The crude product was purified by chromatography (acetone:methylene chloride:hexane =1:2:7) to produce 0.84 g of product (39%). It was mixture of Z and E isomers (Z:E=13:1).

### c. 3α-t-Butyldimethylsiloxy-21-methyl-5α-pregn-17(20)(Z)-ene

The mixture of 5α-3α-hydroxy-21-methylpregn-17(20)(Z)-ene (0.84 g, 2.66 mmol), TBDMSCl (1.2 g, 8.0 mmol) and imidazole (0.91 g, 13.3 mmol) in methylene chloride (10 mL) and DMF (30 mL) was stirred for 12 h and then ammonium chloride was added. It was then extracted with methylene chloride (3x40 mL) and washed with brine (50 mL). The organic solution was dried over potassium carbonate and then solvent removed and it was found that there was still some DMF. It was then redissolved in ether and washed with brine (2x50 mL) and then dried over potassium carbonate. Chromatography with hexane resulted 1.14 g of the pure product (100%).

### d. 3α-t-Butyldimethylsiloxy-20α-hydroxy-21-methyl-5α-pregnane

To a solution of 3α-*t*-butyldimethylsiloxy-21-methyl-5α-pregn-17(20)(Z)-ene (1.14 g, 2.66 mmol) in THF (30 mL) was added diborane THF complex (1M solution in THF, 5.3 mL) dropwise at 0°C. The reaction was allowed to warm to 25 °C for 1 h. Then a solution of sodium hydroxide (20%, 10 mL) was added very slowly at 0°C followed by an addition of hydrogen peroxide (30%, 10 mL). Aqueous ammonium chloride then was added and THF layer was separated by separatory funnel. The aqueous layer was extracted with ether (2x40 mL). The organic solution was dried over potassium carbonate and the solvent removed *in vacuo.* The pure product was obtained by column chromatography (0.52 g, 44%).

### e. 3α,20α-Dihydroxy-21-methyl-5α-pregnane

A solution prepared by mixing HF (48%, 5 mL) and CH₃CN (30 mL) was added to a flask containing 3α-*t*-butyldimethylsiloxy-20α-hydroxy-21-methyl-5α-pregnane (0.51 g) and a white precipitate appeared. The reaction was stirred for 1 h and then filtered. The white solid was washed with ether for three times and it gave satisfactory analytical results (0.3 g, 79%), m.p. 227-231 °C.

### Example 6 (Reference)

### 3α,21-Dihydroxy-3β-trifluoromethyl-5β-19-norpregnan-20-one

To a solution of 3α-hydroxy-3β-trifluoromethyl-5β-19-norpregnan-20-one (300 mg, 0.87 mmol) in toluene (15 mL) was added MeOH (1 mL) and BF₃^{.}OEt₂ (1.4 mL, 11.3 mmol). The resulting mixture was cooled to 0°C and Pb(OAc)₄ (0.54 g, 1.21 mmol) was added. The reaction was warmed to 25°C with stirring for 45 min and then NaHCO₃ solution (sat, 30 ml) was added, and the mixture was stirred for 1 h. It was poured into a separatory funnel containing water (50 mL) and extracted with ether (3x40 mL). The ethereal solution was washed with brine (50 mL) and then dried over K₂CO₃. The crude product obtained by removal of solvent was dissolved in MeOH (25 mL) and K₂CO₃ solution (sat., 8 mL) was added. The reaction was stirred for 5 h and then the reaction mixture was poured into a separatory funnel containing 50 mL of water. It was then extracted with CH₂Cl₂ (3x30 mL). The combined extracts were dried over K₂CO₃ and the crude material obtained was purified by chromatography to give the product (160 mg) along with 21-methoxy by-product (40 mg). The product was further purified by recrystallization from 10% acetone in hexane to give 88 mg of the pure product (28%) as a white solid, m.p. 140-142°C.

### Example 7 (Reference)

### 3β-Ethynyl-3α,20α-dihydroxy-5β-pregnane and 3β-Ethynyl-3α,20β-dihydroxy-5β-pregnane

To a solution of 3β-ethynyl-3α-hydroxy-5β-pregnan-20-one (0.31 g, 0.91 mmol) in methanol (20 mL) was added sodium borohydride (200 mg, 5.3 mmol) and it was stirred at 25°C for 1 h. Ammonium chloride (50 mL) solution was then added and the mixture was extracted with CH₂Cl₂ (3x30 mL). A chromatography (EtOAc:Hex=-3:7) gave 3β-ethynyl-3α,20β-dihydroxy-5β-pregnane (200 mg, 65%) as major product: m.p., 221-223 °C. The minor product, 3β-ethynyl-3α,20β-dihydroxy-5β-pregnane was further purified by another chromatography (25-30% ethyl acetate in hexane, 16 mg, 5%): m.p., 187-188°C.

### Example 8 (Reference)

### 3α,21-Dihydroxy-3β-ethynyl-5β-pregnan-20-one and 3α-Hydroxy-3β-ethynyl-21-methoxy-5β-pregnan-20-one

To a solution of 3α,21-dihydroxy-3β-ethynyl-5β-pregnan-20-one 21-acetate (725 mg, 1.81 mmol) in 45 mL of mehanol at 0°C was added an aqueous 10% K₂CO₃ solution (3.75 mL). After stirring for 30 min at rt, the mixture was recooled to 0°C and a 2N aqueous HOAc solution (1.8 mL) was added. The reaction was added to an EtOAc/water mixture. The aqueous layer was extracted twice with EtOAc and the pooled organic layers were extracted with a brine solution, dried (Na₂SO₄) and concentrated. Purification by flash chromatography (20 cm of silica gel in a 4 cm dia. column, eluted with 2 liters of 20% acetone/hexane) gave 582 mg (90%) of the diol as a white solid, mp 155.5-157°C.

In larger scale preparations of the 3α,21-diol, a less polar impurity was isolated, mp 176-178.5°C and found to be 3α-hydroxy-3β-ethynyl-21-methoxy-5β-pregnan-20-one, probably formed as a by-product in the preparation of the 21-acetate.

### Example 9 (Reference)

### 3β-Ethenyl-3α-hydroxy-5α-pregnan-20-one and 3α-Ethenyl-3β-hydroxy-5α-pregnan-20-one

A solution of 5α-pregnan-3,20-dione, 20-ketal (720 mg, 2 mmol) in dry THF (20 mL) was treated with vinyl magnesium bromide (1M in THF, 4 mmol, 4 mL) at -78 °. After stirring the mixture at this temp. for 5 h, it was quenched with 2N HCl solution (2 mL). The solvent was removed and the residue was then dissolved in acetone (25 mL). After adding 2N HCl (10 mL) the solution was stirred at rt for 15 h. The solvents were removed and the residue was extracted with CH₂Cl₂. The organic layer was washed with water, dil. NaHCO, soln., water, and brine. After drying over anhyd. MgSO₄ the solution was filtered and evaporated to yield the crude product (1 g). This crude product was then dissolved in a small amount of CH₂Cl₂ and poured on a column of silica gel. On elution with toluene:acetone mixture (95:5) gave 3β-ethenyl-3α-hydroxy-5α-pregnan-20-one (250 mg) as a first fraction, mp. 163-165°. Further elution with the same solvent mixture yielded 3α-ethenyl-3β-hydroxy-5α-pregnan-20-one (150 mg).

### Example 10 (Reference)

### 3α-Hydroxy-3β-trifluoromethyl-5β-19-norpregnan-20-one

To a solution of 3α-hydroxy-3β-trifluoromethyl-5α-19-norpregn-17(20)(Z)-ene (2.6 g, 7.3 mmol) in THF (80 ml), was added diborane THF complex (1M solution in THE, 22 mL) dropwise at 25°C. The reaction was complete in 1 h and then a solution of sodium hydroxide (20%, 50 mL) was added very slowly at 0°C followed by an addition of hydrogen peroxide (30%, 30 mL). Water then was added and THF layer was separated by separatory funnel. The aqueous layer was extracted with methylene chloride (2x40 mL). The organic solution was dried over potassium carbonate and the solvent removed *in vacuo.* A quick column (hex:acetone=1:1) resulted 1.8 g product which was subjected to PCC oxidation (PCC, 2.1 g, 9.6 mmol; sodium acetate, 0.8 g, 9.6 mmol). The pure product (700 mg, 26%) was purified by column chromatography using ethyl acetate and hexane (15:85) as eluent; m.p. 151.5-153.0°C.

### Example 11 (Reference)

### a. 3(R)-Spiro-2'-oxirane-5α-17β-hydroxyandrostane

A mixture of trimethylsulfoxonium iodide (6.82 g, 31 mmol) and potassium *t*-butoxide (3.5 g, 31 mmol) in THF (30 mL) was heated to reflux for 1.5 h and then cooled to 25°C. 17β-Hydroxy-5α-androstan-3-one (3 g, 10.3 mmol) was then added and the reaction was stirred at 25 °C for 2 h. Water was then added and the mixture was extracted with ether (3x80 mL). The extract was dried over potassium carbonate and the removal of the solvent resulted in 3 g of fairly pure product (crude yield, 96%) which was used for the next step.

### b. 3β-Methyl-3α-hydroxy-5α-androstan-17-one

To a solution of 3(R)-spiro-2'-oxirane-5α-17β-hydroxyandrostane (3g, 9.9 mmol) in THF (50 mL) under Ar was added lithium aluminum hydride (LAH, 1 M solution in THF, 10 ml) and the mixture was heated to reflux for 5 min and then cooled to 25°C. Ammonium chloride solution (aq., sat., 70 mL) was added and the mixture was then extracted with CH₂Cl₂ (3x70 mL). The organic solution was dried over potassium carbonate and then 4-methylmorpholin-*N*-oxide (2.9 g, 25 mmol) and ground molecular sieves (4A, 10 g) were added. The mixture was then stirred for 20 min and tetrapropylammonium perruthenate (200 mg) was added. The reaction was complete in 1.5 h and the reaction mixture was filtered through Florisil which was rinsed with mixture of CH₂Cl₂ and ether (1:2). The resulted material was purified by chromatography (30% EtoAc in hexane) to produce the product (2.5 g, 83%).

### c. 3β,21-Dimethyl-3α-hydroxy-5α-pregn-17(20)(Z)-ene

To a Wittig reagent prepared by stirring the mixture of n-propyltriphenylphosphonium bromide (2.55 g, 6.6 mmol) and potassium t-butoxide (0.75 g, 6.6 mmol) in THF (20 mL) for 30 min was added 3β-methyl-3α-hydroxy-5α-androstan-17-one (0.5 g, 1.65 mmol) and the mixture was heated to reflux for 18 h. The reaction was then quenched with ammonium chloride solution and extracted with CH₂Cl₂. The pure product (420 mg, 77%) was obtained by chromatography (20% EtOAc in hexane).

### d. 3α,20α-Dihydroxy-3β,21-dimethyl-5α-pregnane

To a solution of 3β,21-dimethyl-3α-hydroxy-5α-pregn-17(20)(Z)-ene (0.42 g, 1.27 mmol) in THF (30 mL) was added diborane THF complex (1M solution in THF, 2.6 mL) dropwise at 0°C. The reaction was allowed to warm to 25 °C for 2 h. Then a solution of sodium hydroxide (2 N, 10 mL) was added very slowly at 0°C followed by an addition of hydrogen peroxide (30%, 10 mL). The reaction was stirred at 25 °C for 1 h. Aqueous ammonium chloride then was added and THF layer was separated by separately funnel. The aqueous layer was extracted with CH₂Cl₂ (2x40 mL). The organic solution was dried over potassium carbonate and the solvent removed *in vacuo.* The product was obtained by column chromatography (0.17 g, 38%). It was further purified by recrystallization to result in 110 mg product m.p., 200-203°C.

### Example 12 (Reference)

### 3α,21 Dihydroxy-3β-trifluoromethyl-5β-pregnan-20-one

A solution of 3α,21-dihydroxy-3β-trifluoromethyl-5β-pregnan-20-one, 21-acetate (1.36g, 3.06 mmol) in MeOH (75mL) was cooled to 0°C. A solution of K₂CO₃ (10% aqueous, 6.45mL, 4.67mmol) was then added dropwise. After stirring for 1.5 hours at 0°C, a solution of acetic acid (2N aqueous, 2.5mL, 5.0 mmol) was added dropwise and the mixture was allowed to warm to room temp. EtOAc, CH₂Cl₂ and water (100mL each) were added and thoroughly mixed. The organic phase was isolated, washed with aqueous NaHCO₃ and NaCl solutions, dried over MgSO₄ and evaporated *in vacuo.* The residue was purified by flash column chromatography (hexane/EtOAc 3:1) to yield a white solid (973 mg, 79%), mp. 148-150°C.

### Example 13 (Reference)

### Sodium 3α,21-dihydroxy-3β-ethynyl-5β-pregnan-20-one 21-hemisuccinate

A solution of 3α,21-dihydroxy-3β-ethynyl-5β-pregnan-20-one (535 mg, 1.49 mmol) in 2 mL of dry pyridine was treated at 0°C with solid succinic anhydride (1.2 eq; 180 mg, 1.80 mmol). After warming to rt, the reaction was allowed to stir for 48 h. Removal of the solvent *in vacuo* and trituration of the residue with hexane (2 x 10 mL) gave a gummy solid which was dissolved as much as possible in CH₂Cl₂ and added to 13 cm of flash silica in a 2 cm dia. column. Elution with a gradient from 5% aeetone/CH₂Cl₂ to 100% acetone gave 667 mg of the desired acid.

To remove pyridinium salts, the acid was dissolved in EtOAc and extracted with an ice-cold 0.01 N aqueous HCl solution (30 mL). The organic layer was dried with Na₂SO₄ and concentrated. The residue, 633 mg, mp 62-68°C, was dissolved in methanol and an aqueous solution of 116 mg (0.253 mmol) of NaHCO₃ was added. After stirring for 3.5 h, the solvent was removed under reduced pressure and the residue was triturated with an ether/hexane mixture. The light yellow solid obtained, weight 616 mg, was soluble in water at > 20 mg/mL.

### Example 14 (Reference)

### 3β-Fluoromethyl-3α-hydroxy-5α-pregnan-20-one

A mixture of *n*-Bu₄NF^{·}xH₂O (7.873 g) and benzene (50 mL) was refluxed with a Dean-Stark trap overnight. The mixture, which was not a clear solution, was then concentrated (normal pressure) to ∼10 mL and allowed to cool to room temperature. A solution of 3(R)-5α-pregnan-3-spiro-2'oxirane-20-one ethyleneketal (2.55 g, 6.81 mmol) in dry benzene (15 mL + 5 mL for the rinse) was added, using a double-ended needle, to the above concentrated solution. The resulting solution was concentrated, using a short-path distillation apparatus, to ∼10 mL and refluxed for 15 min. Since it was very difficult to spot the concentrated reaction solution on the TLC plate, dry benzene (5 mL) was added. TLC (100:1 CH₂Cl₂/acetone or 3:1 hexane/acetone) showed some unreacted starting material, besides two new less-polar spots. The reaction mixture was again concentrated and then refluxed for 30 min; TLC (after diluting the mixture with benzene) now showed almost complete consumption of the starting epoxide. As before, the mixture was concentrated and refluxed for a while. It *must be noted here that this reaction takes place only when the mixture is highly concentrated* After the mixture had come to room temperature (giving a light-yellow solid), ether and water were added. Since all the solid did not dissolve, CH₂Cl₂ was added. The aqueous layer was back extracted with CH₂Cl₂. The combined organic extracts were washed with water (x2), dried (Na₂SO₄), filtered, and evaporated under reduced pressure to provide a white solid (3.33 g) whose ¹H NMR showed it to be a 4:1 mixture of 3β-fluoromethyl-3α-hydroxy-5α-pregnan-20-one ethyleneketal and 3α-fluoro-3β-hydroxymethyl-5α-pregnan-20-one ethylene ketal.

In order to hydrolyze the ketal, acetone (100 mL), water (5 mL), and *p*-TsOH^{·}H₂O (143 mg, 0.752 mmol) were added to the above solid. The pH was adjusted by adding in HCl until slightly acidic. The mixture was heated with a heat gun for a while in order to get a clear solution, which was stirred at room temperature for 2 h. The mixture became turbid, so CH₂Cl₂ was added to obtain a clear solution. TLC indicated completion of the reaction. The solvent was removed under reduced pressure, giving a white solid to which were added CH₂Cl₂ and water. The aqueous layer was back-extracted with CH₂Cl₂. The combined organics were washed with saturated NaHCO₃, dried (MgSO₄), filtered, and evaporated under reduced pressure to furnish a white crystalline solid (2.5 g) whose ¹H NMR showed it to be a 4:1 mixture of 3α-fluoromethyl-3α-hydroxy-5α-pregnan-20-one and 3α-fluoro-3β-hydroxymethyl-5α-pregnan-20-one. Flash column chromatography of this mixture with CH₂Cl₂ as eluent provided the desired 3β-fluoromethyl-3α-hydroxy-5α-pregnan-20-one (1.41 g, 59%), mp 201-203 °C.

### Example 15 (Reference)

### 3β-Ethynyl-3α,20α-dihydroxy-5α-pregnane and 3β-ethynyl-3α,20β-dihydroxy- 5α-pregnane

To a solution of 3β-ethynyl-3α-hydroxy-5α-pregnan-20-one (032 g, 0.94 mmol) in methanol (20 mL) was added sodium borohydride (200 mg, 5.3 mmol) and it was stirred at 25 °C for 1 h. Ammonium chloride (50 mL) solution was then added and the mixture was extracted with CH₂Cl₂ (3x30 mL). Chromatography (EtOAc:Hex=3:7) resulted in 3β-ethynyl-3α,20β-dihydroxy-5α-pregnane (192 mg, 60%) as major product: m.p., 195.5-197.5°C; and 3β-ethynyl-3α,20α-dihydroxy-5α-pregnane as the minor product (19 mg, 6%): m.p., 210-215°C (decomp.).

### Example 16

### 3α,20α-Dihydroxy-21-methyl-5α-pregnane bis hemisuccinate

To a dry flask containing 3α,20α-dihydroxy-21-methyl-5α-pregnane (350 mg, 1.05 mmol) was added pyridine (anhydrous, 5 mL) and then succinic anhydride (1 g, 10 mmol) was added. The mixture was heated in a oil bath at 100°C for 20 h and then cooled to 25°C. It was mixed with HCl solution (1N, 70 mL) and extracted with EtOAc (3x40 mL) and extracts were dried over Na₂SO₄. The product (0.54 g) was obtained by chromatography (7% MeOH and 0.3% HOAc in CH₂CL₂) and was further purified by recrystallization from EtOAc to yield 334 mg of the product (60%), m.p. 159-162.5°C.

### Example 17 (Reference)

### a. 3,3-Ethylenedioxy-5β-pregn-17(20)(Z)-ene

5β-3-Ethylenedioxy-androstan-17-one (6 g) was added to Wittig reagent produced from ethyltriphenylphosphonium bromide (15 g) and potassium *t*-butoxide (4.5 g) in THF (15 mL). The reaction was refluxed for 2 h and cooled to 25°C. Then methylene chloride (80 mL) and a solution of ammonium chloride (60 mL) was added and the organic layer was separated by separatory funnel. The aqueous layer was extracted with methylene chloride (2x50 mL). The organic solution was dried over potassium carbonate and the solvent removed *in vacuo.* Most of the phosphoroxide was removed by washing with hexane. The obtained product (6 g) was dissolved in acetone (100 mL), and hydrochloric acid (2 N, 10 mL) added. The crude product (5.5 g) obtained by a basic work-up followed by methylene chloride extraction was purified by chromatography to pr6duce 4.5 g of product (83%). The pure stereoisomer was obtained by repeated recrystallization from hexane.

### b. 3α-Hydroxy-3β-trifluoromethyl-5β-pregn-17(20)(Z)-ene

To a solution of 5β-pregn-17(20)(Z)-en-3-one (950 mg, 3.17 mmol) in THF (15 mL) was added trifluoromethyltrimethylsilane (0.5 M solution in THF, 9.5 mL) at 0°C. The solution turned to brown gradually and the reaction was finished in 30 min. Water (30 mL) was added and organic layer collected. The aqueous layer was extracted with ether (3x50 mL) and organic solution was dried over potassium carbonate. The pure product (680 mg, 58%) was isolated by column chromatography using ethyl acetate and hexane (1:9) as eluent.

### Example 18 (Reference)

### a. 3α-Hydroxy-5β-pregn-11-en-20-one

To a solution of 5β-pregn-11-en-3,20-dione (Sigma, 15 g, 4.77 mmol) in THF (40 mL) was added lithium tri-*t*-butoxyaluminum hydride (1 M in THF, 5.7 mmol) at -78°C slowly for 15 min. The reaction was allowed to warm to 25 °C for 12 h. The mixture was partitioned between ammonium chloride and ether and the crude material thus obtained was purified by column chromatography (10-20% acetone in hexane, 13 g product, 86%).

### b. 3α-Hydroxy-5β-20,20-ethylenedioxypregn-11-ene

To a mixture of 3α-hydroxy-5β-pregn-11-en-20-one (1.3g), ethylene glycol (8 mL) and trimethylorthoformate (20 mL) was added *p*-toluenesulfonic acid (0.1 g) and the reaction was stirred for 1 h. A normal workup (sodium bicarbonate and ether) followed by the removal of solvent resulted in the crude product which was purified by chromatography (acetone:Hex-1:4, 1.2 g, 81%).

### c. 5β-20,20-Ethylenedioxy-5β-pregn-11-en-3-one

A mixture of 3α-hydroxy-20,20-ethylenedioxy-5β-pregn-11-ene (1.2 g), sodium acetate (0.53 g) and PCC (1.4 g) was stirred at 25°C for 1 h and then filtered through Florisil eluting with methylene chloride and ether (1:2). The pure product was obtained by column chromatography (acetone:hexane=1:9, 0.71 g, 60%).

### d. 3α-Hydroxy-3β-trifluoromethyl-5β-pregn-11-en-20-one

To a solution of 5β-20-ethylenedioxypregn-11-en-3-one (710 mg, 1.98 mmol) in THF (15 mL) was added trifluoromethyltrimethylsilane (0.5 M solution in THF, 6 mL) and then tetrabutylammonium fluoride (20 mg) at 0°C. The solution turned to brown gradually and the reaction was completed in 1 h. Water (30 mL) was added and organic layer collected. The aqueous layer was extracted with ether (3x40 mL) and organic solution was dried over potassium carbonate. The crude product obtained by removing the solvent was hydrolyzed by HCl (2 N, 5 mL) in acetone (40 mL). The pure product (554 mg, 73%) was then isolated by column chromatography using ethyl acetate and hexane (1:5) as eluent m.p., 160-162.5 °C.

### Example 19 (Reference)

### 3α,21-Dihydroxy-3β+ethynyl-5β-pregnan-20-one 21-acetate

A suspension of 3β-ethynyl-3α-hydroxy-5β-pregnan-20-one (1.00 g, 2.92 mmol) in 35 mL of toluene was treated with 2 mL of methanol. The resulting solution was cooled in an ice/water bath and neat BF₃-Et₂O (Aldrich; 5.8 mL, 5.02 g, 35.4 mmol) was added. Solid lead tetraacetate (Aldrich; 1.96 g, 4.42 mmol) was added in a few portions. A light purple solution formed initially and became light brown as stirring continued at 0°C. The mixture was stirred at rt for 3 h and then recooled to 0°C. The cold reaction was added to a mixture of 52 mL of a sat NaHCO₃ solution, water and crushed ice. The resulting mixture was extracted with EtOAc (2 x 75 mL). The combined organic layers were extracted with a sat. NaCl solution, dried (Na₂SO₄) and concentrated. The crude product was purified by column chromatography (25 cm of flash silica gel in a 5 dia. column eluted with 3 liters of 20% acetone/hexane) affording 749 mg (64%) of the acetate, mp 196-198°C.

### Example 20 (Reference)

### 3α,21-Dihydroxy-3β-trifluoromethyl-5β-pregnan-20-one, 21-acetate

Under an anhydrous argon atmosphere, 3α-hydroxy-3β-trifluoromethyl-5β-pregnan-20-one (1.94 g, 5.02 mmol) was dissolved in toluene (86 mL) and MeOH (5.2 mL). Boron trifluoride etherate (10.4 mL, 843 mmol) was added via syringe. Lead tetraacetate (2.89 g, 651 mmol) was then added. The mixture was stirred for 70 min, poured into water and extracted three times with CH₂Cl₂. The organic phases were combined, washed with aqueous NaHCO, and NaCl solutions, dried over MgSO₄ and evaporated in *vacuo* to give a pale yellow solid (2.18 g). This solid was purified by flash column chromatography (CH₂Cl₂/EtOAc 150:1 and hexane/EtOAc 4:1) to give a white solid (1.54g; 69%). mp. 167-168.5°C.

### Example 21 (Reference)

### 3α-Hydroxy-3β-trifluoromethyl-5β-pregnan-20-one

Reference: Krishnamurti, R; Bellew, D. R; Sutya Prakash, G. K. *J. Org*. *Chem.* 1991, *56*, 984.

To solution of 5β-pregnan-3,20-dione 20-ethyleneketal (60 mg, 0.166 mmol) in dry THF (3 mL) was added 0.5M F₃CSi(CH₃)₃ (in THF; 0.5 mL, 0.25 mmol). The resulting colorless solution was cooled to 0°C, and *n*-Bu₄NF^{.}xH₂O (few crystals) was added. Cooling bath was removed, and the mixture was allowed to warm to room temperature. Unlike the same reaction involving 5α-pregnan-3,20-dione 20-ethyleneketal, the above reaction mixture did not turn yellow, and also there was no gas generation. TLC (3:1 hexane/acetone) did not show any product formation. Subsequently, 0.5M F₃CSi(CH₃)₃ (in THF; 0.5 mL, 0.25 mmol) was added. The resulting mixture was stirred at room temperature for few minutes. TLC displayed a new spot that had an R_{f} close to 1, but there was still some unreacted starting material present. Therefore, more 0.5M F₃CSi(CH₃)₃ (in THF; 0.5 mL, 0.25 mmol) was added. The mixture was again stirred at room temperature for a while. No unreacted starting ketone. 1N HCl (∼3 mL) was added, and the resulting two-phase mixture was stirred at room temperature overnight. The spot that was formed as a result of trifluoromethylation had now completely disappeared, and there were two new, less-polar spots, the lower one being the major product. The mixture was then diluted with ether and water. The aqueous layer was separated and extracted with ether. The combined organic layers were washed with saturated NaHCO₃ and brine, dried (MgSO₄), filtered, and evaporated under reduced pressure to give a white crystalline (foamy) solid. ¹H and ¹⁹F NMR of this solid revealed the presence of two epimers in a ratio of 85:15. Separation of these two epimers was achieved by flash chromatography with 15:1 hexane/acetone.

Evaporation of the early fractions gave the minor isomer, which was not characterized and was presumed to be 3β-hydroxy-3α-trifluoromethyl-3β-pregnan-20-one.

Further elution of the column gave 3α-hydroxy-3β-trifluoromethyl-5β-pregnan-20-one (50 mg).

### Example 22 (Reference)

### 3α-Hydroxy-3β-trifluoromethyl-5β-19-norpregn-17(20)(Z)-ene

To a solution of 5β-19-norpregn-17(20)(Z)-en-3-one (823 mg, 2.88 mmol) in THF (30 mL) was added trifluoromethyltrimethylsilane (0.5 M solution in THF, 8.6 mL) at 0°C. The solution turned to brown gradually and the reaction was completed in 30 min. Water (30 mL) was added and the organic layer collected. The aqueous layer was extracted with ether (3x50 mL) and the organic solution was dried over potassium carbonate. The pure product (800 mg, 78%) was isolated by column chromatography using ethyl acetate and hexane (1:9) as eluent.

### Example 23 (Reference)

### 3α,21-Hydroxy-3β-methyl-5α-pregnan-20-one, 21-acetate

A solution of 3α-hydroxy-3β-methyl-5α-pregnan-20-one (3.00 g, 9.02 mmol) in dry toluene (110 mL) and methanol (6 mL) was cooled in an dry-ice/acetone bath to -75°C. Neat BF₃-Et₂O (Aldrich; 18 mL, 146 mmol) was added via syringe, followed by solid lead tetraacetate (4.39 g, 9.90 mmol) added in portions. No reaction was apparent at -75 °C and the reaction was allowed to warm to -10°C over 4 h. The reaction was allowed to warm to 0°C over an additional 90 min. By HPLC, starting material is the major species present. After an hour at 0°C, the reaction was recooled to -15°C and an additional 1.94 g of lead tetraacetate was added, and the reaction was allowed to warm to 0°C. After 30 min, HPLC (scattering detector) gave uncorrected ratio of product and starting material of 10:1, and after an additional 45 min, the reaction was cooled to -10°C and added to an ice-cold mixture of 100 mL of EtOAc,165 mL of a sat. NaHCO₃ solution and crushed ice. The aqueous layer was separated and washed with EtOAc (2 x 150 mL). The pooled organic layers were then washed twice each with water and a sat NaCI solution. After drying over Na₂SO₄, the solvent was removed and the residue was recrystallized from EtOAc, affording 1g of acetate contaminated with starting material. The mother liquor was concentrated *in vacuo* and triturated with two 100 mL portions of hexane. The residue from the hexane washes was combined with the recrystallized material and recrystallized a second time. The resulting solid was contaminated with 2.8 % of starting material (by HPLC). A third recrystallization gave 1.087 g (31% yield) of the acetate which contained <2% of starting material.

### Example 24 (Reference)

### 3α,21-Dihydroxy-5β-pregnan-20-one 21-Disodium phosphate

To a solution of 21-bromo-3α-hydroxy-5β-pregnan-20-one (1.0 g, 2.5 mmol) in 10 mL THF at RT was added dibenzyl hydrogen phosphate (2.1 g, 7.55 mmol) and triethylamine (1.085 mL, 7.8 mmol) with stirring. The reaction was then heated to reflux for 2.5 h and then cooled to RT. Dichloromethane (25 mL) was then added and the solution transferred to a separatory funnel, washed with IN HCl, sat. aq. NaHCO₃, dried with MgSO₄ and concentrated *in vacuo* to give the dibenzylphosphate as a crude oil (943 mg). The oil was dissolved in EtOH (50 mL) and a few drops of sulfuric acid were added. The flask was charged with 400 mg of 5% Pd/C and the stirred solution was then subjected to 1 atm. of H₂ gas at RT until the reaction was complete. The catalyst was removed via filtration and the solution concentrated to a residue. This residue was dissolved in 4 : 1 MeOH: water (20 mL) and titrated to pH 11 with 2N NaOH. Another 50 mL of MeOH was added and upon sitting at 0°C for 1 h solid inorganic phosphate was filtered off. The solution was concentrated *in vacuo* and the residue was washed with hot toluene (∼50 mL) and then dissolved in a minimum volume of MeOH. To this solution acetone was slowly added until a solid precipitated. The mixture was centrifuged, the solvent decanted off and the wet solid transferred to a vial and dried under vacuum to yield the title compound as a hydroscopic solid.

### Example 25 (Reference)

### 3α,21-Dihydroxy-3β-methyl-5α-pregnan-20-one 21-Disodium phosphate

To a solution of 21-bromo-3α-hydroxy-3β-methyl-5α-pregnan-20-one (1.0 g, 2.43 mmol) in 10 mL THF at RT was added dibenzyl hydrogen phosphate (2.1 g, 7.3 mmol) and triethylamine (1.085 mL, 7.53 mmol) with stirring. The reaction was then heated to reflux for 4.5 h and then cooled to RT. Dichloromethane (25 mL) was then added and the solution transferred to a separatory funnel, washed with IN HCl, sat. aq. NaHCO₃, dried with MgSO₄ and concentrated *in vacuo* to give the dibenzylphosphate as a crude oil (1.205 g). The dibenzylphosphate (790 mg, 1.3 mmol) was dissolved in 2 : 1 EtOH : THF (30 mL) with a few drops of sulfuric acid, charged with 5% Pd/C (180 mg, 20% by weight) and subjected to 50 psi of H₂ at rt until the reaction was complete by TLC. The catalyst was removed by filtration and the filtrate was concentrated. The residue was dissolved in 4 : 1 MEOH : water (10 mL) and titrated to pH 11 with 1M NaOH. The solution was treated with acetone until a readily filterable solid precipitated after which the mixture was cooled to 0°C and filtered to isolate a crude solid 260 mg. The solid was dissolved in 20 mL water forming a cloudy solution that was filtered and then concentrated to give 220 mg of the title compound as a white solid.

### Example 26 (Reference)

### a. 20,20-Ethylenedioxy-5α-pregnan-3-one

The title compound was prepared in about 90% overall yield starting from 3β-hydroxy-5α-pregnan-20-one, using the method described for the 5β compound.

### b. 3β-Ethynyl-3α-hydroxy-5α-pregnan-20-one, 20-ketal and 3α-Ethynyl-3β-hydroxy-5α-pregnan-20-one, 20-ketal

A 250 mL three neck flask equipped with a gas inlet, a thermometer and a condenser was charged with lithium acetylide-EDA complex (2.75 g, 90%, 27.5 mmol). Dry benzene (60 mL) was added and acetylene gas was bubbled through the mixture at a moderate rate. The mixture was then heated to 50-55°C in an oil bath and treated in parts with 5a-pregnan-3,20-dione, 20-ketal (9 g, 25 mmol). The stirring was continued at this temp. for 5 h and then at rt for another 17 h. The resulting suspension was cooled to 10°C and was treated with sat. NaCI soln. (5 mL). The solvent was removed and the residue was taken up in water. The water insoluble product was collected by filtration, washed with water, and dried under vacuum. This crude product was then recrystallized from EtOAc to yield 3α-ethynyl-3β-hydroxy-5α-pregnan-20-one, 20-ketal (3.35 g). The mother liq. was evaporated to dryness and the residue was purified by column chromatography over silica gel. Elution with a toluene:acetone mixture (92:8) gave the unreacted starting ketone (1.3 g) followed by 3β-ethynyl-3α-hydroxy-5α-pregnan-20-one, 20-ketal (1.3 g) as a second fraction. Further elution with the same solvent mixture yielded the more polar 3α-ethynyl-3β-hydroxy-5α-pregnan-20-one, 20-ketal (270 mg).

### c. 3β-Ethynyl-3α-hydroxy-5α-pregnan-20-one

3β-Ethynyl-3α-hydroxy-5α-pregnan-20-one, 20-ketal (550 mg) was dissolved in a mixture of acetone (20 mL) and 2N HCl (10 mL) and the mixture was stirred at rt for 15 h. The solvents were removed and the residue was extracted with CH₂Cl₂. The organic layer was washed with water, dil. NaHCO₃ soln., water, and brine. After drying over anhyd. MgSO₄ the solution was filtered and evaporated to yield the crude product (414 mg). This crude product was then dissolved in a small amount of CH₂Cl₂ and poured on a column of silica gel. Elution with toluene:acetone mixture (92:8) gave the title compound (280 mg), mp 175-177°C.

### d. 3α-Ethynyl-3β-nitrooxy-5α-pregnan-20-one

A solution of 3α-ethynyl-3β-hydroxy-5α-pregnan-20-one, 20-ketal (2.15 g) in CHCl₃ (45 mL) was cooled to -20° and treated with acetic anhydride (20 mL). Fuming nitric acid (4 mL) was then added and the mixture was stirred at this temp. for 45 min. After warming to -5°, the yellow solution was poured into a mixture of 2N NaOH (70 mL) and water (150 mL) to yield the resultant solution of pH 3-4. It was then extracted with CHCl₃, washed with water, sat NaHCO₃ solution, brine, dried (MgSO₄) and evaporated to yield the title compound as a viscous material (3 g), which was used as such for the next step.

### e. 3β-Ethynyl-3α-hydroxy-5α-pregnan-20-one and 3α-Ethynyl-3β-hydroxy-5α-pregnan-20-one

The crude product (3 g) from above step was taken in a mixture of THF and water (30 mL, 1:1) and AgNO₃ (516 mgl was added. After stirring at rt for 15 h the solvents were removed and the residue was extracted with CH₂Cl₂. The organic layer was washed with water, dil. NaHCO₃ soln, water, and brine. After drying over anhyd. MgSO₄ the solution was filtered and evaporated to yield the crude product (2 g). This crude product was then dissolved in a small amount of CH₂Cl₂ and poured on a column of silica gel. Elution with a toluene:acetone mixture (93:7) gave 3β-ethynyl-3α-hydroxy-5α-pregnan-20-one (550 mg) as a first fraction. Further elution with the same solvent mixture yielded the more polar 3α-ethynyl-3β-hydroxy-5α-prognan-20-one (460 mg).

### Example 27 (Reference)

### 3α,21-Dihydroxy-3β-trifluoromethyl-5β-pregnan-20-one, 21-hemisuccinate

Under an anhydrous argon atmosphere, 3α,21-dihydroxy-3β-trifluoromethyl-5β-pregnan-20-one (920 mg, 2.29 mmol), succinic anhydride (457 mg, 4.57 mmol) and dimethylaminopyridine (14 mg) were dissolved in anhydrous pyridine. After stirring for 16 hours, more dimethylaminopyridine (7 mg) was added. After stirring for 3 hours more, the solvent was removed in *vacuo.* Residual pyridine was removed by evaporation from a toluene solution. The residue was purified by flash column chromatography (CH₂Cl₂/MeOH; gradient 100:1 to 50:1) to yield a white semi-solid residue (1.1 g). This slightly impure solid was dissolved in CH₂Cl₂, washed with water (3 x 50 mL) and dried over MgSO₄. The solvent was removed in *vacuo* to yield a white solid (883 mg, 77%).

### 3α,21-Dihydroxy-3β-trifluoromethyl-5β-pregnan-20-one, 21-hemisuccinate, sodium salt

3α,21-Dihydroxy-3β-trifluoromethyl-5β-pregnan-20-one, 21-hemisuccinate (859 mg, 1.71 mmol) was dissolved in MeOH (35 mL). NaHCO₃ (143 mg, 1.70 mmol) in water (10 mL) was then added dropwise. More MeOH (10 mL) was added. After stirring for 2.5 hours, the solvent was evaporated. Toluene was added and evaporated *in vacuo.* The residue was rubbed with ether/hexane and then with hexane to give a white solid. MeOH was added and removed. Heptane was added and removed *in vacuo.* The residue was rubbed with hexane and dried *in vacuo* to give a white solid (878 mg, 98%).

### Example 28 (Reference)

### 3β-Ethynyl-3α-hydroxy-5β-pregn-11-en-20-one

To a solution of cis/trans dibromoethylene (236 mg, 1.27 mmol) in 5 mL dry THF at -78°C was added BuLi 1.6M in hexanes (1.6 mL, 2.54 mmol) dropwise and the mixture stirred for 30 minutes. The temperature was then lowered to -90°C and a solution of 5β-pregn-11-en-3,20-one in 10 mL THF was added via cannula over 10 min and the reaction stirred at -90°C for another 30 min after which 3 mL of sat. aq. NH₄Cl was added and the mixture stirred to RT. The solvent level was reduced under vacuum to ∼5 mL and then partitioned between ethylacetate and water (25 mL each), and the organic layer was washed with sat. aq. NaCl, dried with MgSO₄ and concentrated *in vacuo to* give a crude solid. Flash chromatography on 6 in. of silica gel in a 2 cm column collecting 10 mL fractions eluting with 95 : 5 Toluene: Acetone resulted in 142 mg (65.6%) of the title compound. MP 147-50°C.

### Example 29 (Reference)

### 3β-Trifluoromethyl-3α-hydroxy-5α-19-norpregnan-20-one

This compound was obtained along as a by-product in the preparation of 3α-hydroxy-3β-trifluoromethyl-5β-19-norpregnan-20-one (*see,* Example 10). It is isolated from the appropriate chromatographic fractions: m.p., 178-179°C.

### Example 30 (Reference)

### 3β-(1-Heptynyl)-3α-hydroxy-5β-pregnan-20-one

A solution of 1-heptyne (0.327 mL, 2.5 mmol) in dry THF (15 mL) was treated with n-BuLi (2.5M in THF, 2.5 mmol, 1 mL) at -78°C. After stirring the mixture at this temperature for 1 hr, a solution of 5β-pregnan-3,20-dione cyclic 20-(1,2-ethanediyl acetal) (360 mg, 1 mmol) in dry THF (15 mL) was added and the mixture was stirred at -78°C for 1 hr. It was then quenched with 2N HCl solution (1 mL). The solvent was removed and the residue was then dissolved in acetone (10 mL). After adding 2N HCl (10 mL) the solution was stirred at rt for 1 hr. Saturated NaHCO₃ soln. was added to neutralize the acid. The solvents were removed and the residue was extracted with EtOAc. The organic layer was washed with water, dil. NaHCO₃ soln., water, and brine. After drying over anhyd. MgSO₄ the solution was filtered and evaporated to yield the crude product (400 mg), This crude product was then dissolved in a small amount of CH₂Cl₂ and poured on a column of silica gel. Elution with toluene:acetone mixture (93:7) gave 3β-(1-heptynyl)-3α-hydroxy-3β-pregnan-20-one (260 mg) as a colorless solid; mp 121-123°C.

An analogous method was used to prepare: 3β-(1-hexynyl)-3α-hydroxy-5β-pregnan-20-one, 3β-(1-octynyl)-3α-hydroxy-5β-pregnan-20-one, 3β-cyclopropylethynyl-3α-hydroxy-5β-pregnan-20-one, 3β-(3-methylbut-2-en-1-ynyl)-3α-hydroxy-5β-pregnan-20-one, and 3β-(3,3-dimethylbutynyl)-3α-hydroxy-5β-pregnan-20-one.

### Example 31

### 3β-Cyclopropylethynyl-3α-hydroxy-5β-pregnan-20-one

### a. 3β-(5-Chloro-1-pentynyl)-3α-hydroxy-5β-pregnan-20-onecyclic 20-(1,2-ethanediyl acetal)

A solution of 5-chloropentyne (0.5 mL, 4.8 mmol) in dry THF (15 mL) was treated with n-BuLi (2.4M in THF, 4.8 mmol, 2 mL) at -60°. After stirring the mixture at -78°C for 0.5 hr, a solution of 5β-pregnan-3,20-dione, cyclic 20-(1,2-ethanediyl acetal) (560 mg, 1.56 mmol) in THF (15 mL) was added and the mixture was stirred at -78°C for 1 hr. The cooling bath was removed and the mixture was quenched with NH₄Cl solution (3 mL). The solvent was removed and the residue was extracted with EtOAc. The organic layer was washed with water, dil. NaHCO₃ soln., water, and brine. After drying over anhyd. MgSO₄ the solution was filtered and evaporated to yield the crude product (660 mg). This crude product was used as such for the next step.

### b. 3β-Cyclopropylethynyl-3α-hydroxy-5β-pregnan-20-one

A solution of diisopropylamine (0.4 mL, 3 mmol) in dry THF (15 mL) was treated with n-BuLi (2.5M in THF, 3 mmol, 1.2 mL) at -60°. After stirring the mixture at -78°C for 0.5 hr, a solution of 3β-(5-chloro-1-pentynyl)-3α-hydroxy-5β-pregnan-20-one cyclic 20-(1,2-ethanediyl acetal) (100 mg, 0.22 mmol) in THF (15 mL) was added. The cooling bath was removed and the mixture was stirred at rt for 0.5 hr. It was then quenched with NH₄Cl solution (3 mL). The solvent was removed and the residue was then dissolved in acetone (40 mL). After adding 1N HCl (4 mL) the solution was stirred at r.t. for 15 min. Sat. NaHCO₃ soln. was added to neutralize the acid. The solvents were removed and the residue was extracted with EtOAc. The organic layer was washed with water, dil. NaHCO₃ soln., water, and brine. After drying over anhyd. MgSO₄ the solution was filtered and evaporated to yield the crude product (120 mg). This crude product was then dissolved in a small amount of CH₂Cl₂ and poured on a column of silica gel. Elution with toluene:acetone mixture (95:5) gave 3β-(cyclopropylethynyl)-3α-hydroxy-5β-pregnan-20-one (55 mg) as a colorless solid; mp 123-138°C; TLC R_{f} (hexane:acetone 7:3) 0.29

It will be obvious to one skilled in the art that the above described compounds may be present as mixtures of diastereomers which may be separated into individual diastereomers. Resolution of the diastereomers may be conveniently accomplished by gas or liquid chromatography or isolation from natural sources. Unless otherwise specified herein, reference in the specification and claims to the compounds of the invention, as discussed above, is intended to include all isomers, whether separated or mixtures thereof.

Where isomers are separated, the desired pharmacological activity will often predominate in one of the diastereomers. As disclosed herein, these compounds display a high degree of stereospecificity. In particular, those compounds having the greatest affinity for the GABA receptor complex are those with 3β-substituted-3α-hydroxypregnane steroid skeletons.

The compounds of and used in the invention, that being the nontoxic, pharmaceutically acceptable, natural and synthetic, direct acting and "prodrug" forms of progesterone, deoxycorticosterone, and androstane metabolites, have hitherto unknown activity in the brain at the GABA_{A} receptor complex. The present invention takes advantage of the discovery of this previously unknown mechanism and activity.

The pharmaceutical compositions of this invention are prepared in conventional dosage unit forms by incorporating an active compound of the invention or a mixture of such compounds, with a nontoxic pharmaceutical carrier according to accepted procedures in a nontoxic amount sufficient to produce the desired pharmacodynamic activity in a subject, animal or human. Preferably, the composition contains the active ingredient in an active, but nontoxic amount, selected from about 1 mg to about 500 mg of active ingredient per dosage unit. This quantity depends on the specific biological activity desired and the condition of the patient. Desirable objects of the compositions and methods of this invention are in the treatment of stress, anxiety, PMS, PND, and seizures such as those caused by epilepsy to ameliorate or prevent the attacks of anxiety, muscle tension, and depression common with patients suffering from these central nervous system abnormalities. An additional desirable object of the composition and methods is to treat insomnia and produce hypnotic activity. Another desirable object of the compounds an methods is to induce anesthesia, particularly by intravenous administration.

The pharmaceutical carrier employed may be, for example, either a solid, liquid, or time release (see e.g. Remington's Pharmaceutical Sciences, 14th Edition, 1970). Representative solid carriers are lactose, terra alba, sucrose, talc, gelatin, agar, pectin, acacia, magnesium stearate, stearic acid, microcrystalline cellulose, polymer hydrogels and the like. Typical liquid carriers are propylene glycol, glycofurol, aqueous solutions of cyclodextrins, syrup, peanut oil, and olive oil and the like emulsions. Similarly, the carrier or diluent may include any time-delay material well known to the art, such as glycerol monostearate or glycerol distearate alone or with wax, microcapsules, microspheres, liposomes, and/or hydrogels.

A wide variety of pharmaceutical forms can be employed. Thus, when using a solid carrier, the preparation can be plain milled micronized, in oil, tableted, placed in a hard gelatin or enteric-coated capsule in micronized powder or pellet form, or in the form of a troche or lozenge. The compounds of and used in the invention may also be administered in the form of suppositories for rectal administration. Compounds may be mixed in material such as cocoa butter and polyethylene glycols or other suitable non-irritating material which is solid in room temperature but liquid at the rectal temperature. When using a liquid carrier, the preparation can be in the form of a liquid, such as an ampule, or as an aqueous or nonaqueous liquid suspension. Liquid dosage forms also need pharmaceutically acceptable preservatives and the like. In addition, because of the low doses that will be required as based on the data disclosed herein, parental administration, nasal spray, sublingual and buccal administration, and timed release skin patches are also suitable pharmaceutical forms for topical administration.

The method of producing anxiolytic, anticonvulsant, mood altering (such as anti-depressant) or hypnotic activity, in accordance with this invention, comprises administering to a subject in need of such activity a compound of the invention, usually prepared in a composition as described above with a pharmaceutical carrier, in a nontoxic amount sufficient to produce said activity.

During menses, the levels of excreted metabolites vary approximately fourfold (Rosciszewska *et al.,* 1986). Therefore, therapy for controlling symptoms involves maintaining the patient at a higher level of progesterone metabolites than normal in the premenstrual state of PMS patients. Plasma levels of active and major metabolites are monitored during pre-menses and postmenses of the patient. The amount of the compounds of the invention administered, either singly or as mixtures thereof are thus calculated to reach a level which will exert GABA-receptor activity equal or higher than the level of progesterone metabolites in the normal subject during the premenses state.

The route of administration may be any route that effectively transports the active compound to the GABA_{A} receptors that are to be stimulated. Administration may be carried out parenterally, enterally, rectally, intravaginally, intradermally, intramuscularly, sublingually, or nasally; the oral, intramuscular and dermal routes are preferred. For example, one dose in a skin patch may supply the active ingredient to the patient for a period of up to one week. However, the parenteral route is preferred for status epilepticus.

### Potency and Efficacy at the GR Site

The *in vitro* and in *vivo* experimental data show that the naturally-occurring metabolites of progesterone/deoxycorticosterone and their derivatives interact with high affinity at a novel and specific recognition site on the GR complex to facilitate the conductance of chloride ions across neuronal membranes sensitive to GABA (Gee *et al.,* 1987; Harrison *et al.,* 1987).

To those skilled in the art, it is known that the modulation of [³⁵S]t-butylbicyclophosphorothionate ([³⁵S]TBPS) binding is a measure of the potency and efficacy of drugs acting at the GR complex, which drugs may be of potential therapeutic value in the treatment of stress, anxiety, and seizure disorders (Squires, R.F., *et al.,* "[³⁵S]t-Butylbicyclophophorothionate binds with high affinity to brain-specific sites coupled to a gamma aminobutyric acid-A and ion recognition site," *Mol, Pharmacol.,* 23:326, 1983; Lawrence, L.J., *et al.,* "Benzodiazepine anticonvulsant action: gamma-aminobutyric acid-dependent modulation of the chloride ionophore," *Biochem. Biophys. Res. Comm.,* 123:1130-1137, 1984; Wood, *et al., "In vitro* characterization of benzodiazepine receptor agonists, antagonists, inverse agonists and agonist/antagonists," *Pharmacol, Exp. Ther., 231*:572-576, (1984)). We performed several experiments to determine the nature of the modulation of [³⁵S]TBPS as affected by the compounds of the invention. We found that these compounds interact with a novel site on the GR complex which does not overlap with the barbiturate, the benzodiazepine or any other previously known sites. Furthermore, these compounds have high potency and efficacy at the GR complex, with stringent structural requirements for such activity.

The procedures for performing this assay are fully discussed in: (1) Gee, *et al.,* 1987.; and (2) Gee, K.W., L.J. Lawrence, and H.I. Yamamura, *Molecular Pharmacology, 30*:218 (1986). These procedures were performed as follows:

Brains from male Sprague-Dawley rats were removed immediately following sacrifice and the cerebral cortices dissected over ice. A P₂ homogenate was prepared as previously described (Gee *et al.,* 1986). Briefly, the cortices were gently homogenized in 0.32 M sucrose followed by centrifugation at 1000 x g for 10 minutes. The supernatant was collected and centrifuged at 9000 x g for 20 minutes. The resultant P₂ pellet was suspended as a 10% (original wet weight/volume) suspension in 50 mM Na/K phosphate buffer (pH 7.4) 200 mM NaCI to form the homogenate.

One hundred microliter (ml) aliquots of the P₂ homogenate (0.5 milligrams (mg) protein) were incubated with 2 nanomolar (nM) [³⁵S]TBPS (70-110 curies/millimole;, New England Nuclear, Boston, MA) in the presence or absence of the naturally occurring steroids or their synthetic derivatives to be tested. The tested compounds were dissolved in dimethylsulfoxide (Baker Chem. Co., Phillipsbury, NJ) and added to the incubation mixture in 5 µL aliquots. The incubation mixture was brought to a final volume of 1 mL with buffer. Nonspecific binding was defined as binding in the presence of 2 mM TBPS. The effect and specificity of GABA (Sigma Chem. Co., St. Louis, MO) was evaluated by performing all assays in the presence of GABA plus (+)bicuculline (Sigma Chem. Co.). Incubations maintained at 25°C for 90 minutes (steady state conditions) were terminated by rapid filtration through glass fiber filters (No. 32, Schleicher and Schuell, Keene, NH). Filter-bound radioactivity was quantitated by liquid scintillation spectrophotometry. Kinetic data and compound/[³⁵S]TBPS dose-response curves were analyzed by nonlinear regression using a computerized iterative procedure to obtain rate constants and IC₅₀ (concentration of compound at which half-maximal inhibition of basal [³⁵S]TBPS binding occurs) values.

The experimental data obtained for this assay are also published in Gee *et al*., 1987. The data discussed in this reference are shown and described in PCT published application no. W093/03732, published on 04 March 1993. In contrast, the steroid binding site identified by this work is distinct from the GABA/bicuculline site. The shift in dose-response curves induced by bicuculline when the inhibition of [³⁵S]TBPS binding is caused by alphaxalone is not parallel. This indicates that the GABA and steroid sites do not overlap.

A second set of experiments were performed to demonstrate that steroids, barbiturates and benzodiazepines do not share common binding site on the GABA receptor. The assay was performed in accordance with the procedures outlined above. The resulting kinetic data show that the dissociation of [³⁵S] TBPS binding initiated by saturating concentration of 3α-hydroxy-5α-pregnan-20-one is potentiated by 100 µM Na pentobarbital. This effect is an indication that 3α-OH-5a-pregnan-20-one (steroid) and pentobarbital (barbiturate) bind to independent sites.

The third set of experiments examined the interactions between 3α-hydroxy-5α-pregnan-20-one and Na pentobarbital in the potentiation of (³H) flunitrazepam (FLU) binding. These experiments further support the claim that steroids do not share common site of action with benzodiazepines and barbiturates. In this series of experiments, the effect of varying concentrations of 3α-hydroxy-5α-pregnan-20-one on (³H) FLU binding in the presence or absence of a maximally stimulating concentration of Na pentobarbital. Since Na pentobarbital has greater maximum efficacy than that of 3α-hydroxy-5α-pregnan-20-one in potentiating (³H)FLU binding, 3α-hydroxy-5α-pregnan-20-one should ultimately antagonize the effect of Na pentobarbital if the two interact competitively on the same site. This is not what was observed. Thus, the data further support our conclusion that certain steroids including the compounds of and used in the invention interact with a novel site distinct from the barbiturate or BZ regulatory site on the GR complex. Because of this independent site of action, it is anticipated that these steroid compounds will have therapeutic profiles different from those of barbiturates and BZs.

Various compounds were screened to determine their potential as modulators of [³⁵S]TBPS binding *in vitro.* These assays were performed in accordance with the above discussed procedures. Based on these assays, we have established the structure-activity requirements for their specific interaction at the GR complex and their rank order potency and efficacy. Table I provides IC₅₀ and maximum inhibition of several compounds, including examples of those claimed in the application. IC₅₀ is defined as concentration of compounds to inhibit 50% of control [³⁵S]TBPS binding. It is an indication of a compound's *in vitro* potency. Maximum inhibition is an indication of a compound's *in vitro* efficacy.

**Table 1**

| | IC₅₀ | I_{MAX} |
|---|---|---|
| 3β-cyclopropylethynyl-3α-hydroxy-5β-pregnan-20-one | 22 | 94 |
| 3α-hydroxy-3β-(3'-methyl-but-3'-en-1'-ynyl)-5β-pregnan-20-one | 24 | 91 |
| 3α-hydroxy-3β-methyl-5β-19-nor-pregnan-20-one | 26 | 94 |
| 3α,20α-dihydroxy-21-ethyl-5α-pregnane | 27 | 13 |
| 3β-(cyclopropyl)ethynyl-3α-hydroxy-5β-pregnan-20-one | 32 | 97 |
| 3α,20α-dihydroxy-21-methyl-5α-pregnane | 35 | 28 |
| 3α-hydroxy-5α-pregnan-20-one | 37 | 95 |
| 3α-hydroxy-3β-methyl-5β-pregnan-20-one | 37 | 98 |
| 3β-ethynyl-3α-hydroxy-5β-pregnan-20-one | 39 | 101 |
| 3β-ethynyl-3α-hydroxy-19-nor-5β-pregnan-20-one | 44 | 93 |
| 3β-(5'-chloropentyn-1'-yl)-3α-hydroxy-5β-pregnan-20-one | 44 | 98 |
| 3β-ethenyl-3α-hydroxy-5β-pregnan-20-one | 46 | 100 |
| 3α-hydroxy-3β-ethynyl-5α-pregnan-20-one | 53 | 91 |
| 3α-hydroxy-3β-methyl-5α-pregnan-20-one | 62 | 97 |
| Bis (3α,21-dihydroxy-3β-ethynyl-5β-pregnan-20-one) 21-hemisuccinate | 68 | 56 |
| 3α,21-dihydroxy-5α-pregnan-20-one 21-acetate | 75 | 99 |
| 3α,21-dihydroxy-5α-pregnan-20-one (5α-THDOC) | 76 | 100 |
| 3α-Hydroxy-3β-trifluoromethyl-19-nor-5β-pregnan-20-one | 76 | 100 |
| 3α-hydroxy-21-methyl-5β-pregnan-20-one | 78 | 100 |
| 3α-hydroxy-3β-(3'-methyl-but-3'-en-1'-ynyl)-5α-pregnan-20-one | 88 | 95 |
| 3α-hydroxy-3β,21-dimethyl-5α-pregnan-20-one | 89 | 85 |
| 3α-hydroxy-3β-methyl-5α-19-nor-pregnan-20-one | 91 | 80 |
| 3α,20a(S)-dihydroxy-5α-pregnane | 99 | 46 |
| N-(3α-hydroxy-3β-methyl-5α-pregnan-20-ylidine)ethanolamine | 101 | 93 |
| 3β-ethenyl-3α-hydroxy-5α-pregnan-20-one | 113 | 88 |
| 3α,21-dihydroxy-5α-pregnan-20-one 21-hemisuccinate | 117 | 91 |
| 3α,21-dihydroxy-5β-pregnan-20-one 21-acetate | 119 | 49 |
| 3α,20α-dihydroxy-3β-methyl-5α-pregnane | 166 | 52 |
| 3α,20-dihydroxy-20-methyl-5α-pregnane | 176 | 40 |
| 3α,21-dihydroxy-3β-ethynyl-5β-pregnan-20-one 21 hemisuccinate | 179 | 82 |
| Bis (3α,21-dihydroxy-3β-trifluoromethyl-5β-pregnan-20-one) 21-hemisuccinate | 189 | 23 |
| 3α-hydroxy-3β-fluoromethyl-5α-pregnan-20-one | 200 | 77 |
| 3α,20α-dihydroxy-3β-ethynyl-5α-pregnane | 202 | 56 |
| 3α-hydroxy-3β-trifluoromethyl-5β-pregn-11-en-20-one | 203 | 99 |
| 3α,21-dihydroxy-3β-methyl-5α-pregnan-20-one 21-Hemisuccinate, sodium salt | 211 | 104 |
| 3α-hydroxy-3β-trifluoromethyl-5β-pregnan-20-one | 211 | 98 |
| 3α,21-dihydroxy-3β-ethynyl-5β-pregnan-20-one | 216 | 104 |
| 3α,21-dihydroxy-3β-methyl-5α-pregnan-20-one 21-hemisuccinate | 241 | 92 |
| 3α-hydroxy-3β-methyl-5α-pregn-11-en-20-one | 242 | 76 |
| 3α-hydroxy-3β-trifluoromethyl-5α-pregnan-20-one | 244 | 44 |
| 3α,21-dihydroxy-3β-trifluoromethyl-19-nor-5β-pregnan-20-one | 246 | 100 |
| 3α,21-dihydroxy-5α-pregnan-20-one 21-hemisuccinate, sodium salt | 251 | 92 |
| 3α-hydroxy-3β-trifluoromethyl-19-nor-5α-pregnan-20-one | 251 | 40 |
| 3α,21-dihydroxy-3β-ethynyl-5β-pregnan-20-one, 21-acetate | 251 | 101 |
| 3α,21-dihydroxy-3β-methyl-5α-pregnan-20-one | 258 | 96 |
| 3α,21-dihydroxy-3β-methyl-5α-pregnan-20-one 21-acetate | 284 | 93 |
| 3α,21-dihydroxy-3β-ethenyl-5α-pregnan-20-one | 288 | 101 |
| 3α,21-dihydroxy-3β-trifluoromethyl-5β-pregnan-20-one | 354 | 102 |
| 3α,21-dihydroxy-3β-trifluoromethyl-5β-pregnan-20-one, 21-acetate | 370 | 88 |
| 3α,21-dihydroxy-3β-ethenyl-5α-pregnan-20-one 21-hemisuccinate | 377 | 89 |
| 3α,20β-dihydroxy-3β-methyl-5β-pregnane | 455 | 105 |
| 3α,20α-dihydroxy-3β,21-aimethyl-5α-pregnane | 507 | 48 |
| 3α,21-dihydroxy-3β-trifluoromethyl-19-nor-5β-pregnan-20-one, 21-hemisuccinate, sodium salt | 530 | 101 |
| 3α,21-dihydroxy-3β-ethynyl-5β-pregnan-20-one 21 hemisuccinate sodium salt | 540 | 102 |
| 3α,21-dihydroxy-3β-trifluoromethyl-5β-pregnan-20-one 21 hemifumarate sodium salt | 546 | 102 |
| 3α,20α-dihydroxy-3β-ethynyl-5β-pregnane | 557 | 98 |
| 3α,21-dihydroxy-3β-fluoromethyl-5α-pregnan-20-one | 592 | 101 |
| 3α,21-dihydroxy-3β-trifluoromethyl-5β-pregnan-20-one, 21-hemisuccinate | 648 | 101 |
| 3α,20β-dihydroxy-3β-ethynyl-5β-pregnane | 688 | 77 |
| 3α,21-dihydroxy-3β-trifluoromethyl-5β-pregnan-20-one, 21-hemisuccinate, sodium salt | 712 | 99 |
| 3α,20β-dihydroxy-3β-ethynyl-5α-pregnane | 774 | 107 |
| 3α,21-dihydroxy-3β-trifluoromethyl-5β-pregnan-20-one methyl 21-succinate | 480 | 86 |
| 3α-hydroxy-3β-methyl-21,21,21-Trifluoromethyl-5α-pregnan-20-one | 915 | 101 |
| 3α,20β-dihydroxy-3β-methyl-5α-pregnane | 924 | 99 |
| 3α-hydroxy-3β-methyl-5β-pregn-16-en-20-one | 955 | 100 |
| 3α-hydroxy-3β-methyl-5α-pregnane-11,20-dione | 958 | 100 |
| 21-bromo-3α-hydroxy-3β-trifluoromethyl-5β-pregnan-20-one | 1530 | 102 |
| estradiol | > 1000 | I* |
| progesterone | > 1000 | I* |
| corticosterone | > 1000 | I* |
| cholesterol | > 1000 | I* |

| | | |
|---|---|---|
| * = Inactive. | | |

As can be seen from Table 1, compounds of and used in the invention have low IC₅₀, which is the concentration necessary to achieve 50% maximal inhibition of [³⁵S]TBPS binding, while compounds such as sex steroids (estradiol and progesterone), glucocorticoids (corticosterone) and cholesterol having a high IC₅₀ are essentially inactive. Thus, it is anticipated that hormonal steroids and cholesterol per se will not have any therapeutic value for the indications described herein. In order to distinguish this unique class of steroids from hormonal steroids, they are now termed neuroactive steroids. However, sex steroids such as progesterone can be metabolized in the body to steroids similar to 3α-hydroxy-5α-pregnan-20-one. Thus, progesterone can be considered as a prodrug. The TBPS data correlates well with data on ³⁶Cl ion uptake-potentiated by various 3α-hydroxylated steroids described in Purdy R.H., *et al., J. Med. Chem 33*:1572-1581 (1990), incorporated herein by reference and these data also correlate well with electrophysiological data obtained by measuring steroid's activity to potentiate GABA-induced current in oocyte injected with human GABA receptors. This indicates that the TBPS assay is an approximate measurement of steroids ability to allosterically modulate Cl⁻ channel activity.

### Compounds with Partial Activity

In as much as the desired therapeutic activity should be available to the patient with the least undesirable side effects, a notable aspect of this invention involves the discovery of agonists with partial activity in those compounds with a 5α-pregnan-3α,20α-hydroxyl, 5β-pregnan-3α,20β-hydroxyl group or the derivatives and prodrugs of these compounds. In addition, a subset of neuroactive steroids other than these two groups also show partial efficacy in TBPS assay (Table 1). For the patients who desire amelioration of anxiety or convulsions, hypnosis is undesired. For the patients who desire amelioration of insomnia, anesthetic is undesired. The compounds and activities described as agonists with partial activity expected to the desired effect with minimal undesired effect.

In addition, the ability of these compounds to potentiate GABA-mediated enhancement of Cl⁻ current was limited as compared to that of 3α-hydroxy-5α-pregnan-20-one in Xenopus oocyte injected with human GABA_{A} receptor genes.

When Xenopus oocyte expression system was used to test the limit efficacy property of some neuroactive steroids, the following procedure was performed. Xenopus laevis oocytes (stage VI) which had been "defolliculated" using the collagenase digestion method [3 hrs @ 18-23 °C 2 mg ml⁻¹ collagenase 'A' in Barth's saline with Ca²⁺ salts omitted) were injected with cRNA transcripts of human GABA_{A} receptor subunit complex α1 β1 and γ1. The major GABA_{A} receptor complex is comprised of αβγ subunits. Injected oocytes were individually maintained in 96-well plates (200 µL per well of normal Barth's solution supplemented with penicillin 50 IU ml⁻¹, streptomycin 50 mg ml⁻¹ and gentomycin 100 mg ml⁻¹) for up to 9 days at 19-20°C. Agonist-induced currents were recorded from Xenopus oocytes voltage clamped at a holding potential of -60 mV, using an Axoclamp 2 A (Axon Instruments) voltage clamp amplifier in the twin electrode voltage clamp mode. The voltage-sensing and current-passing microelectrodes were filled with 3M KCl, and resistances of 1-3 M ohams when measured in the standard extracellular saline. The oocytes were continuously superfused with frog Ringer (120 mM NaCl; 2 mM KCl; 1.0 mM CaCl₂; 5 mM HEPES pH 7.4) at the rate of 5-7 ml min⁻¹ at room temperature (17-21°C).

All drugs were applied via the perfusion system. Steroids (10⁻² M) were prepared as concentrated stock solutions either in DMSO or ethanol and then diluted in the Ringer solution at the appropriated concentration. The final DMSO and ethanol concentration was 0.2% v/v, a concentration which had no effect upon GABA evoked responses. Stock solutions of all other drugs were made in Ringer solution. Membrane current responses were low-pass filtered at 100 Hz and recorded onto magnetic tape using an FM tape recorder (Racal Store 4DS) for subsequent analysis.

### Benefits over Progesterone

The correlations between reduced levels of progesterone and the symptoms associated with PMS, PND, and catamenial epilepsy (Backstrom, *et al.,* 1983; Dalton, K., 1984) led to the use of progesterone in their treatment (Mattson, *et al.,* 1984; and Dalton, 1984). However, progesterone is not consistently effective in the treatment of the aforementioned syndromes. For example, no dose-response relationship exists for progesterone in the treatment of PMS (Maddocks, *et al.,* 1987). These results are predictable when considered in light of the results of our *in vitro* studies which demonstrate that progesterone has very low potency at the GR complex, as seen in Table 1, compared to certain metabolites of progesterone.

The beneficial effect of progesterone is probably related to the variable conversion of progesterone to the active progesterone metabolites. The use of specific progesterone metabolites in the treatment of the aforementioned syndromes is clearly superior to the use of progesterone based upon the high potency and efficacy of the metabolites and their derivatives (See Gee, *et al.,* 1987, and Table 1 above).

### No Hormonal Side Effects

It has also been demonstrated that neuroactive steroids lack hormonal side effects by the lack of affinity for the progesterone and other hormonal steroid receptors. The data were obtained by performing assays in accordance with the procedures described in Gee, *et al.* 1988 previously to determine the effect of progesterone metabolites and their derivatives and the progestin R5020 on the binding of [³H]R5020 to the progesterone receptor in rat uterus (Gee *et al.* 1988).

³H-progesterone (0.15 nM) was incubated with the rat uterus cytosol in the presence of the test compounds. The specific bindings were determined after incubation and compared to the control incubation without the compounds. The results are expressed as percent inhibition of binding. If the compounds bind to the progesterone receptor with high affinity, a 100% inhibition of binding would be expected at the concentration tested. Neuroactive steroids exhibit less than 10% inhibition indicating that they are inactive as progestenic agents.

Various hormonal activities of representative neuroactive steroids were further studied through testing their potential estrogenic, mineralocorticoid and glucocorticoid activities. These activities were analyzed by monitoring the ability of the compounds to inhibit binding of the steroid hormones to their respective hormone receptors. The results of these experiments are reported in PCT published application W093/03732, published on 04 March 1993.

The results of these experiments clearly show that neuroactive steroids do not have a strong affinity for any of the above steroid receptors. Thus, they will not have predicted hormonal side-effects which would result from such steroid receptor bindings.

### Anti-Convulsant Activity

Experiments were also performed to determine the physiological relevance of neuroactive steroid and GABA receptor interactions by assessing the ability of the compounds of and used in the invention to prevent metrazol induced convulsions in mice. Mice were injected with various doses of the test compounds of the invention, 10 minutes prior to the injection of metrazol. The time to onset of myoclonus (presence of forelimb clonic activity) induced by metrazol was determined by observing each mouse for a period of 30 minutes. In control mice, metrazol (85 mg/kg) will induce convulsion in 95% of the animals. The ability of several compounds of and used in the invention to protect mice from convulsion is shown in Table 2.

**Table 2:**

| ***Antimetrazol Activity of Neuroactive Steroids in Mice*** | | | | |
|---|---|---|---|---|
| Name | Route | Vehicle | Dose mg/kg | % Protected |
| 3β-Cyclopropylethynyl-3α-hydroxy-5β-pregnan-20-one | IP | 50% hpbcd | 10 | 87.5 |
| 3α-Hydroxy-3β-(3'-methyl-but-3'-en-1'-ynyl)-5β-pregnan-20-one | IP | 50% hpbcd | 10 | 100 |
| 3α-Hydroxy-5α-pregnan-20-one | IP | 50% hpbcd | 10 | 100 |
| 3α-Hydroxy-3β-methyl-5β-pregnan-20-one | IP | micro soln. | 10 | 70 |
| 3β-Ethynyl-3α-hydroxy-5β-pregnan-20-one | IP | 50% hpbcd | 10 | 100 |
| 3β-Ethynyl-3α-hydroxy-19-nor-5β-pregnan-20-one | IP | 50% hpbcd | 10 | 87.5 |
| 3β-(1-Hexynyl-3α-hydroxy-5β-pregnan-20-one | IP | 50% hpbcd | 10 | 37.5 |
| 3β-Ethenyl-3α-hydroxy-5β-pregnan-20-one | IP | 50% hpbcd | 10 | 87.5 |
| 3α-Hydroxy-3β-ethynyl-5α-pregnan-20-one | IP | 50% hpbcd | 10 | 87.5 |
| 3α-Hydroxy-3β-methyl-5α-pregnan-20-one | IP | 50% hpbcd | 10 | 100 |
| Bis (3α,21-dihydroxy-3β-ethynyl-5β-pregnan-20-one) 21-hemisuccinate | IP | 50% hpbcd | 10 | 37.5 |
| 3α,21-Dihydroxy-5α-pregnan-20-one 21-acetate | IP | 50% hpbcd | 10 | 50 |
| 3α,21-Dihydroxy-5α-pregnan-20-one (5α-THDOC) | IP | 50% hpbcd | 10 | 87.5 |
| 3α-Hydroxy-3β-trifluoromethyl-19-nor-5β-pregnan-20-one | IP | 50% hpbcd | 10 | 100 |
| 3β-Ethynyl-3α-hydroxy-5β-pregn-11-en-20-one | IP | 50% hpbcd | 10 | 75 |
| 3α-Hydroxy-3β-(3'-methyl-but-3'-en-1'-ynyl)-5α-pregnan-20-one | IP | 50% hpbcd | 10 | 87.5 |
| 3α-Hydroxy-3β-methyl-5α-19-nor-pregnan-20-one | IP | micro soln. | 10 | 37.5 |
| 3α,20a(S)-Dihydroxy-5α-pregnane | IP | micro soln. | 10 | 30 |
| 3β-Ethenyl-3α-hydroxy-5α-pregnan-20-one | IP | 50% hpbcd | 10 | 100 |
| 3α,21-Dihydroxy-5β-pregnan-20-one 21-acetate | IP | 50% hpbcd | 10 | 25 |
| 3α,20a-Dihydroxy-3β-methyl-5α-pregnane | IP | micro soln. | 100 | 60 |
| 3α,21-Dihydroxy-3β-ethynyl-5β-pregnan-20-one 21 hemisuccinate | IP | water | 10 | 50 |
| Bis (3α,21-dihydroxy-3β-trifluoromethyl-5β-pregnan-20-one) 21 -hemisuccinate | IP | 50% hpbcd | 10 | 25 |
| 3α-Hydroxy-3β-fluoromethyl-5α-pregnan-20-one | IP | 50% hpbcd | 10 | 14.3 |
| 3α,21-Dihydroxy-3β-methyl-5α-pregnan-20-one 21-Hemisuccinate, sodium salt | IP | micro soln. | 10 | 11.11 |
| 3α-Hydroxy-3β-trifluoromethyl-5β-pregnan-20-one - | IP | 50% hpbcd | 10 | 62.5 |
| 3α,21-Dihydroxy-3β-ethynyl-5β-pregnan-20-one | IP | 50% hpbcd | 10 | 18.8 |
| 3α,21-Dihydroxy-3β-methyl-5α-pregnan-20-one 21-Hemisuccinate | IP | water | 10 | 37.5 |
| 3α-Hydroxy-3β-trifluoromethyl-5α-pregnan-20-one | IP | 50% hpbcd | 10 | 62.5 |
| 3α,21-Dihydroxy-3β-trifluoromethyl-19-nor-5β-pregnan-20-one | IP | 50% hpbcd | 10 | 118.8 |
| 3α,21-Dihydroxy-5α-pregnan-20-one 21-hemisuccinate, sodium salt | IP | water | 60 | 100 |
| 3α-Hydroxy-3β-trifluoromethyl-19-nor-5α-pregnan-20-one | IP | 50% hpbcd | 10 | 25 |
| 3α,21-Dihydroxy-3β-ethynyl-5β-pregnan-20-one, 21-acetate | IP | 50% hpbcd | 10 | 62.5 |
| 3α,21-Dihydroxy-3β-methyl-5α-pregnan-20-one | IP | 50% hpbcd | 10 | 87.5 |
| 3α,21-Dihydroxy-3β-methyl-5α-pregnan-20-one 21-acetate | IP | micro soln. | 10 | 10 |
| 3α,21-Dihydroxy-3β-trifluoromethyl-5β-pregnan-20-one | IP | 50% hpbcd | 10 | 50 |
| 3α,21-Dihydroxy-3β-trifluoromethyl-5β-pregnan-20-one, 21-acetate | IP | 50% hpbcd | 10 | 62.5 |
| 3α-Hydroxy-3β-trifluoromethyl-5β-19-nor-pregn-17(Z)-ene | IP | 50% hpbcd | 10 | 25 |
| 3α,20-Dihydroxy-3β,20-dimethyl-5β-pregnane | IP | micro soln. | 10 | 22.2 |
| 3α,20α-Dihydroxy-3β,21-dimethyl-5α-pregnane | IP | dmso | 10 | 0 |
| 3α,21-Dihydroxy-3β-trifluoromethyl-19-nor-5β-pregnan-20-one, 21-hemisuccinate, sodium salt | IP | water | 10 | 50 |
| 3α,21-Dihydroxy-3β-ethynyl-5β-pregnan-20-one 21 hemisuccinate sodium salt | IP | water | 10 | 50 |
| 3α,21-Dihydroxy-3β-trifluoromethyl-5β-pregnan-20-one 21 hemifumarate sodium salt | IP | water | 10 | 50 |
| 3α,21-Dihydroxy-3β-fluoromethyl-5α-pregnan-20-one | IP | 50% hpbcd | 10 | 25 |
| 3α,21-Dihydroxy-3β-trifluoromethyl-5β-pregnan-20-one, 21-hemisuccinate | IP | water | 10 | 50 |
| 3α,20β-Dihydroxy-3β-ethynyl-5β-pregnane | IP | 50% hpbcd | 10 | 25 |
| 3α,21-Dihydroxy-3β-trifluoromethyl-5β-pregnan-20-one, 21-hemisuccinate, sodium salt | IP | water | 10 | 62.5 |
| 3α,20β-Dihydroxy-3β-ethynyl-5α-pregnane | IP | 50% hpbcd | 10 | 37.5 |

The ability of neuroactive steroids to protect animals against other chemical convulsants was further demonstrated for 3α-hydroxy-5α-pregnan-20-one, 3α,21-dihydroxy-5α-pregnan-20-one and 3α-hydroxy-3β-methyl-5β-pregnan-20-one. The anticonvulsant tests are similar to that described above. The following chemical convulsants are: metrazol (85 mg/kg); (+)bicuculline (2.7 mg/kg); picrotoxin (3.15 mg/kg); strychnine (1.25 mg/kg); or vehicle (0.9% saline). Immediately after the injection of convulsant or vehicle, the mice were observed for a period of 30 to 45 minutes. The number of animals with tonic and/or clonic convulsions was recorded. In the maximal electroshock test, 50 mA of current at 60 Hz was delivered through corneal electrodes for 200 msec to induce tonic seizure. The ability of compounds to abolish the tonic component was defined as the endpoint. General CNS depression potential was determined by a rotorod test 10 minutes after the injection of compounds where the number of mice staying on a rotating (6 rpm) rod for 1 minute in one of the three trials was determined. The ED₅₀ (the dose at which the half-maximal effect occurs) was determined for each screen and are presented in Table 3, *infra.* The results demonstrate that neuroactive steroids, in comparison to other clinically useful anti-convulsants, are highly effective with profiles similar to that of the BZ clonazepam. These observations demonstrate the therapeutic utility of these compounds as modulators of brain excitability, which is in correspondence with their high affinity interaction with the GR complex *in vitro.*

**Table 3.**

| Anticonvulsant Activity of Exemplified Neuroactive Steroids and Selected Known Anticonvulsants in Mice | | | | | | |
|---|---|---|---|---|---|---|
| **ED**_{**50**} **(mg/Kg)** | | | | | | |
| **Compound** | **RR** | **MES** | **MTZ** | **BIC** | **PICRO** | **STR** |
| 3α5α^{(a)}-P | 30 | 28.6 | 4.9 | 12.3 | 10.2 | >300 |
| 5α-THDOC^{(a)} | 22.9 | 26.7 | 8.1 | 17.8 | 5.6 | >300 |
| 3α-hydroxy-3β-methyl-5α-pregnan-20 one^{(b)} | 246.2 | >100 | 6.3 | 61.9 | 35.4 | >100 |
| Clonazepam* | 0.184 | 93 | 0.009 | 0.0086 | 0.043 | NP |
| Phenobarbital* | 69 | 22 | 13 | 38 | 28 | 95 |
| Phenytoin* | 65 | 10 | NP | NP | NP | ** |
| Progabide*** | -- | 75 | 30 | 30 | 105 | 75 |
| Valproate* | 426 | 272 | 149 | 360 | 387 | 293 |

| | | | | | | |
|---|---|---|---|---|---|---|
| The abbreviations are RR (Rotorod); MES (maximal electroshock); MTZ (metrazol); BIC (bicuculline); PICRO (picrotoxin); STR (strychnine); NP (no protection) ^{(a)} Dissolved in 20% hydroxypropyl-β-cyclodextrin in water. The route of administration for steroids and convulsants was i.p. and s.c., respectively. | | | | | | |
| * Anticonvulsant data are from Swinyard & Woodhead, General principles: experimental detection, quantification and evaluation of anticonvulsants, in *Antiepileptic Drugs,* D.M. Woodbury, J.K. Penry, and C.E. Pippenger, eds. p. 111, (Raven Press, New York), 1982. | | | | | | |
| ^{(b)} Vehicle contained 0.32% hydroxypropylmethyl cellulose and 4% TWEEN 80 in saline. | | | | | | |
| ** Maximum protection of 50% at 55-100 mg/kg. | | | | | | |
| *** The chemical convulsants in the progabide studies were administered i.v., all data from Worms *et al.,* Gamma-aminobutyric acid (GABA) receptor stimulation. I. Neuropharmacological profiles of progabide (SL 76002) and SL 75102, with emphasis on their anticonvulsant spectra, *Journal of Pharmacology and Experimental Therapeutics 220:* 660-671 (1982). | | | | | | |

### Anxiolytic Effects

The following experiments demonstrate that the progesterone metabolites, 3α-OH-5α-pregnan-20-one and 3α-OH-5β-pregnan-20-one are effective anxiolytics in four animal models of human anxiety that measure the behavioral effects of anxiolytic compounds. It is to be understood that these two compounds describe the invention by way of illustration. Data on their synthetic derivatives in these measurements also is presented in Tables 4-6. The four animal models used to measure the behavioral effects of anxiolytic compounds are: 1) light/dark transition test; 2) elevated plus-maze; 3) Geller-Seifter conflict test and 4) Vogel test.

### a) Light/dark Transition Test

The light/dark transition test (Crawley and Goodwin, *Pharmacol. Biochem. Behav. 13*:67-70 1980) is based on the observation that rodents naturally tend to explore novel environments, but open, brightly lit arenas are aversive to the rodents and inhibit exploratory behavior (Christmas and Maxwell, *Neuropharmacol. 9*:17-29 1970; File, *J. Neurosci. Meth. 2*:219-238 1980). A variety of clinically established anxiolytics including diazepam, clonazepam and pentobarbital have been shown to increase the number of transitions between the light box and the dark box, whereas nonanxiolytic drugs do not demonstrate this behavioral effect (Crawley *et al., Neuropharmacol. 23*:531-537 (1984)).

Male N.I.H. Swiss-Webster mice (Harlan, Harlan, Indianapolis, IN) weighing 15-20 g were housed four per cage in polyethylene cages with sawdust bedding. The colony room was environmentally controlled (22°C) with a 12 hr light/dark cycle (0600-1800 hr). Food and water were available *ad libitum,* except during testing. The experiments were run from 0700-1500 hr and groups were counterbalanced for time of day effects. Mice were only administered drug or vehicle once.

The method used was a modification of methods previously described (Wieland *et al., Br. Res. 565*:263-268 (1991)). The apparatus included two 2-compartment automated test chambers (Model RXYZCM16, Omnitech Electronics, Columbus, OH). The open compartment was connected to the enclosed compartment via a 7.5 x 7.5 cm passageway. The open compartment was brightly lit using a 200 W incandescent light bulb. The experimental room was kept dark. Interruptions of the infrared beams in either chamber were automatically recorded by being linked to a computer through a Digiscan Analyzer (Omnitech Electronics) and the data was analyzed using the Integrated Lab Animal Monitoring System (Omnitech Electronics).

N.I.H. Swiss-Webster mice were administered vehicle or test drug intraperitoneally (IP), 10 min later they were placed in the center of the lit compartment. The number of transitions between the lit and dark chambers, total activity in the lit chamber and the time spent in the lit chamber were measured during a 10 min test period.

Administration of 3α-OH-5α-pregnan-20-one and 3α-OH-5β-pregnan-20-one in the light/dark transition test produced a significant dose-response curve in relation to the number of transitions between the dark box and the light box. Post-hoc comparisons showed that the number of the crossing for doses for both 3α-OH-5α-pregnan-20-one and 3α-OH-5β-pregnan-20-one were significantly increased at doses tested from control (Dunnett's t-test).

In addition both 3α-OH-5α-pregnan-20-one and 3α-OH-5β-pregnan-20-one produced significant (p < 0.01) increases in activity at 10 & 20 mg/kg as compared to control groups (Dunnett's t-test). There were no significant differences between the two compounds at any dose tested.

### b) Elevated Plus-Maze

The theoretical basis for the elevated plus-maze test is similar to that of the light/dark transition test. As described by Pellow *et al., J. Neurosci. Meth 14*:149-167 (1985), the elevated plus-maze apparatus is designed to utilize the mice's natural aversion to open spaces. The apparatus consists of two open-arms and two enclosed-arms. The elevated plus-maze test allows for two measures of anxiety, the number of entries into the open-arms and the time spent on the open-arms, both expressed as a percentage of the total number of entries and time spent in/on both the open-arms and enclosed-arms.

Male N.I.H. Swiss-Webster mice (Harlan, Indianapolis, IN) weighing 15-20 g were housed four per cage in polyethylene cages with sawdust bedding. The colony room was environmentally controlled (22°C) with a 12 hr light/dark cycle (0600-1800 hr). Food and water were available *ad libitum,* except during testing. The experiments were run from 0700-1500 hr and groups were counterbalanced for time of day effects. Mice were only administered drug or vehicle once.

The method used was previously described (Lister, *Psychopharmacol. 92*:180-185 (1987)). The apparatus included two open arms perpendicular to two enclosed arms elevated 50 cm from the floor. Each arm was 50 cm long and the walls of the enclosed arms were 40 cm tall. The maze was made completely of black plexiglass. Incandescent 200 W light bulbs were above each of the open arms to produce a strong contrast between the open arms and the enclosed arms.

Ten minutes after an injection, the N.I.H. Swiss-Webster mice were placed in the center of the plus-maze facing an open arm. During the 5 min test period, the number of entries onto the open arms and the enclosed arms, and the time spent in the open arms and enclosed arms were measured. All four paws had to be within an arm for the dependent variable to be measured. Therefore, the time spent in the center of the maze is not counted, so the total time spent in the open arms and the enclosed arms may not equal 5 min.

Both 3α-OH-5α-pregnan-20-one and 3α-OH-5β-pregnan-20-one, in the elevated plus-maze test, demonstrate increased proportion of entries into the open-arms across doses. 3α-OH-5α-pregnan-20-one produced significant increase in entries at 20 mg/kg (p ≤ 0.05), whereas 3α-OH-5β-pregnan-20-one produced significant increases in entries at 5 mg/kg (p ≤ 0.05), 7.5 mg/kg (p ≤ 0.01), and 10 mg/kg (p ≤ 0.01).

In addition, 3α-OH-5α-pregnan-20-one and 3α-OH-5β-pregnan-20-one produced dose-dependent increases in the time spent in the open-arms. 3α-OH-5α-pregnan-20-one produced significant increases in time spent on the open-arms at 10 mg/kg (p ≤ 0.01), whereas 3α-OH-5β-pregnan-20-one produced significant increases in time spent on the open-arms at 7.5 mg/kg (p ≤ 0.01) and 10 mg/kg (p ≤ 0.01).

The results of these experiments are reported in PCT published application WO93/03732, published on 04 March 1993. Table 4 shows the summary of anxiolytic activities of compounds of and used in the invention using the elevated plus-maze under the same conditions described above.

**Table 4.**

| ***Anxiolytic Activity in Plus Maze in Mice*** | | | | |
|---|---|---|---|---|
| Name | Route | Vehicle | Dose mg/kg | % Control |
| 3α-Hydroxy-3β-(3'methyl-but-3'-en-1'-ynyl)-5β-pregnan-20-one | IP | 50% hpbcd | 10 | 155 |
| 3α-Hydroxy-5α-pregnan-20-one | IP | 50% hpbcd | 20 | 143.7 |
| 3β-Ethynyl-3α-hydroxy-5β-pregnan-20-one | IP | 50% hpbcd | 1 | 186.6 |
| 3β-Ethynyl-3α-hydroxy-19-nor-5β-pregnan-20-one | IP | 50% hpbcd | 10 | 172 |
| 3β-Ethenyl-3α-hydroxy-5β-pregnan-20-one | IP | 50% hpbcd | 10 | 151 |
| 3α-Hydroxy-3β-ethynyl-5α-pregnan-20-one | IP | 50% hpbcd | 10 | 213 |
| 3α-Hydroxy-3β-methyl-5α-pregnan-20-one | IP | 50% hpbcd | 5 | 165.6 |
| 3α,21-Dihydroxy-5α-pregnan-20-one 21-acetate | IP | 50% hpbcd | 10 | 133 |
| 3α-Hydroxy-3β-trifluoromethyl-19-nor-5β-pregnan-20-one | IP | 50% hpbcd | 10 | 173.8 |
| 3β-Ethynyl-3α-hydroxy-5β-pregn-11-en-20-one | IP | 50% hpbcd | 10 | 233 |
| 3α-Hydroxy-3β-(3'-methyl-but-3'-en-1'-ynyl)-5α-pregnan-20-one | IP | 50% hpbcd | 10 | 141 |
| 3β-Ethenyl-3α-hydroxy-5α-pregnan-20-one | IP | 50% hpbcd | 5 | 154.3 |
| 3α,20a-Dihydroxy-3β-methyl-5α-pregnane | IV | micro soln. | 10 | 174.5 |
| 3α,21-Dihydroxy-3β-ethynyl-5β-pregnan-20-one 21 hemisuccinate | IP | 50% hpbcd | 10 | 164.2 |
| Bis (3α,21-dihydroxy-3β-trifluoromethyl-5β-pregnan-20-one) 21-hemisuccinate | IP | 50% hpbcd | 10 | 92 |
| 3α-Hydroxy-3β-fluoromethyl-5α-pregnan-20-one | IP | 50% hpbcd | 20 | 150.7 |
| 3α-Hydroxy-3β-trifluoromethyl-5β-pregnan-20-one | IP | 50% hpbcd | 20 | 197.6 |
| 3α,21-Dihydroxy-3β-ethynyl-5β-pregnan-20-one | IP | 50% hpbcd | 10 | 162 |
| 3α,21-Dihydroxy-3β-trifluoromethyl-19-nor-5β-pregnan-20-one | IP | 50% hpbcd | 10 | 140 |
| 3α-Hydroxy-3β-trifluoromethyl-19-nor-5α-pregnan-20-one | IP | 50% hpbcd | 10 | 161 |
| 3α,21-Dihydroxy-3β-ethynyl-5β-pregnan-20-one, 21-acetate | IP | 50% hpbcd | 10 | 159 |
| 3α,21-Dihydroxy-3β-trifluoromethyl-5β-pregnan-20-one | IP | 50% hpbcd | 10 | 237 |
| 3β-Ethynyl-3α-hydroxy-5β-pregnan-11,20-dione | IP | 50% hpbcd | 5 | 235.9 |
| 3α,21-Dihydroxy-3β-trifluoromethyl-5β-pregnan-20-one, 21-acetate | IP | 50% hpbcd | 5 | 143.3 |
| 3α-Hydroxy-3β-trifluoromethyl-5β-pregnan-11,20-dione | IP | 50% hpbcd | 10 | 112 |
| 3α,20a-Dihydroxy-3β,21-dimethyl-5α-pregnane | IP | dmso | 10 | 73 |
| 3α,21-Dihydroxy-3β-trifluoromethyl-19-nor-5β-pregnan-20-one, 21-hemisuccinate, sodium salt | IP | water | 20 | 161.1 |
| 3α,21-Dihydroxy-3β-ethynyl-5β-pregnan-20-one 21 hemisuccinate sodium salt | PO | water | 10 | 159 |
| 3α,21-Dihydroxy-3β-trifluoromethyl-5β-pregnan-20-one 21 hemifumarate sodium salt | IP | water | 50% hpbcd | 126 |
| 3α,21-Dihydroxy-3β-fluoromethyl-5α-pregnan-20-one | IP | 50% hpbcd | 10 | 109 |
| 3α,20-Dihydroxy-3β-ethynyl-5β-pregnane | IP | dmso | 10 | 178 |
| 3α,21-Dihydroxy-3β-trifluoromethyl-5β-pregnan-20-one, 21-hemisuccinate, sodium salt | IP | water | 20 | 172.8 |

### c) Geller-Seifter Conflict Test

This animal model of human anxiety utilizes a conditioned state of conflict in rats to ascertain the anxiolytic properties of drugs. Rats are conditioned to bar press for positive reinforcement under two schedules of behavior (Geller and Seifter, *Psychopharmacologia 1*:482-492 (1960)). The first includes bar pressing under a variable ratio schedule without punishment. The second component is a fixed ratio schedule with each bar press resulting in a positive reinforcement and a punishment. The punished component produces a state of conflict within the animal. The unpunished component allows for the observation of any response depressant effects a drug may possess. An anxiolytic response would increase the punished responding without affecting the unpunished responding.

Male albino Sprague-Dawley rats (Charles River Labs, Wilmington, MA) weighing 250-300 g were used for conflict experiments and were kept on a restricted diet of Purina Lab Chow food pellets with water available at all times to maintain body weight at 85% of their free-feeding young adult levels. Rats were housed individually under a 12-hour light-dark cycle with lights on from 0700-1900.

The anti-anxiety (punishment-lessening) and response depressant effects of 3α-OH-5α-pregnan-20-one and 3α-OH-5α-pregnan-20-one were measured in rats by the conflict test of Geller and Seifter (1960). In this 63-min test, hungry rats perform a lever-press response to obtain a sweetened milk reward. The reinforcement schedule consists of punishment and nonpunishment components, alternating approximately every 15 min. Rats were trained in test chambers (Coulbourn instruments) with a lever mounted in one wall, a small dipper that delivered the 0.1-mL milk reward (1 part Eagle condensed : milk 2 parts water), and a metal grid floor through which the foot-shock punishment was administered. A DEC PDP 11/73 minicomputer running SKED (State Systems) was used for programming and recording.

Rates initially learned to respond on a continuous reinforcement schedule and progressed rapidly to 30-sec, 1-min, and 2-min variable interval (VI) schedules. On the continuous reinforcement schedule, rats received milk reward following every lever press; on the VI schedules, milk rewards were available at infrequent and variable intervals, eventually at an average of once every 2 min. Four 3-min "conflict" periods were then introduced on the unpunished VI baseline; the first started after 3 min of VI performance and the others were alternated between 12-min periods of VI responding. During conflict periods, which were signalled by the presentation of a light and a tone, the continuous reinforcement schedule was again in force and each lever press delivered both a milk reward and a brief (0.25 msec) foot-shock punishment. Shock intensity was 0.2 mA initially, and.was increased daily in increments of 0.02 mA in order to gradually suppress lever pressing to 5 responses or less per conflict period. This training took 4-6 weeks, after which stable low rates of response were observed during conflict periods and stable high rates in the nonpunishment periods. Drug-induced increases in the rate of punished responses were taken as an index of antianxiety activity, while decreases in the rate of unpunished responses were taken as an index of response depression or sedation.

The effects of 3α-OH-5α-pregnan-20-one and 3α-OH-5β-pregnan-20-one in the conflict test are summarized herein. Both compounds produced large increases in the rate of punished responses, suggesting that both would be active as antianxiety agents. The peak effect of 3α-OH-5β-pregnan-20-one was observed at 2 mg/kg and that of 3α-OH-5α-pregnan-20-one at 4.4 mg/kg following subcutaneous administration. (For statistical analysis and because of the small number of tests at each dose, all tests with each compound were combined for comparison against vehicle control tests, using a t-test for related measures: for 3α-OH-5α-pregnan-20-one, p < 0.02; for 3α-OH-5β-pregnan-20-one, p < 0.008).

Table 5 shows the summary of anxiolytic activities of compounds of and used in the invention using Geller-Seifter test under the experimental conditions described above.

**Table 5.**

| **Anxiolytic Activity in Geller/Seifter in Rats** | | | | |
|---|---|---|---|---|
| **Compounds** | **Route** | **Vehicle** | **Dose (mg/kg)** | **Geller/Seifter (% of control)** |
| 3α-Hydroxy-5β-pregnan-20-one | sc | 50% hpbcd | 2 | 860.5 |
| 3α-Hydroxy-5α-pregnan-20-one | sc | 50% hpbcd | 5 | 471.8 |
| 3β-Ethynyl-3α-hydroxy-5β-pregnan-20-one | sc | 50% hpbcd | 2 | 751.1 |
| 3α-Hydroxy-3β-ethynyl-5α-pregnan-20-one | sc | 50% hpbcd | 8 | 830.2 |
| 3α-Hydroxy-3β-methyl-5α-pregnan-20-one | sc | 50% hpbcd | 4 | 1384.6 |
| 3α-Hydroxy-3β-trifluoromethyl-19-nor-5β-pregnan-20-one | sc | 50% hpbcd | 4 | 1073.1 |
| 3β-Ethenyl-3α-hydroxy-5α-pregnan-20-one | po | 50% hpbcd | 12 | 1092.3 |
| 3α-Hydroxy-3β-fluoromethyl-5α-pregnan-20-one | sc | 50% hpbcd | 8 | 1005 |
| 3α-Hydroxy-3β-trifluoromethyl-5β-pregn-11-en-20-one | sc | 50% hpbcd | 4 | 508.3 |
| 3α-Hydroxy-3β-trifluoromethyl-5β-pregnan-20-one | sc | 50% hpbcd | 4 | 663.2 |
| 3α,21-Dihydroxy-3β-trifluoromethyl-5β-pregnan-20-one | sc | 50% hpbcd | 16 | 982.4 |
| 3α-Hydroxy-3β-trifluoromethyl-5β-19-norpregn-17(20)-ene | sc | 50% hpbcd | 16 | 1130.3 |

| Prodrugs | Route | Vehicle | Dose (mg/kg) | Geller/Seifter (% of control) |
|---|---|---|---|---|
| 3α,21-Dihydroxy-3β-trifluoromethyl-5β-pregnan-20-one, 21-acetate | sc | 50% hpbcd | 8 | 882.8 |
| 3α,21-Dihydroxy-3β-ethynyl-5β-pregnan-20-one 21-hemisuccinate | po | water | 16 | 1350.0 |
| 3α,21-Dihydroxy-3β-trifluoromethyl-5β-pregnan-20-one 21-hemisuccinate, sodium salt | po | water | 8 | 840.0 |
| 3α,21-Dihydroxy-3β-trifluoromethyl-5β-19-nor-pregnan-20-one 21-hemisuccinate, sodium salt | po | water | 8 | 2493.4 |
| 3α-Hydroxy-3β-methyl-5α-pregnane-20-spiro-2'-(1'3'-thiazolidine-4'-carboxylic acid) | sc | 50% hpbcd | 16 | 900.0 |

### d) Vogel Test

The Vogel test is based on the development of a conflict between a highly motivated behavior and an aversion. For this test, the strong motivation is thirst. The animal is water deprived for 12-16 hrs to produce the motivation to drink. During the training period the animals are exposed to the testing environment so they become accustomed to the drinking spout and minimize the fear of a novel environment. Following training, the animals are allowed access to water for 2 hrs. During this time, the animals will drink and eat their normal amount of water and food, compensating for the deprivation time. However, this schedule still produces a strong motivation to drink during the testing period.

Having been deprived of water for twelve to sixteen hours, an animal is placed in a test cage where it is allowed to drink freely for five minutes. This period is used to habituate the animal to the environment and the drinking spout Following the training period, animals have access to water and food in their home cage for 2 hrs. Food is available at all times. Twenty-four hours later, drug is administered to the animal intracerebroventricularly.

After an indicated delay started from the time of injection the animal is again placed in the test cage for ten minutes. A computer counts each time the animal licks, and after every twentieth lick administers a mild electric stimulus across the tongue and/or feet. The electrical stimulus consists of a 0.6 mA current with a duration of 100 msec. This procedure produces a state of conflict for the animal that is reduced by the administration of clinically used anxiolytic agents (i.e., Valium). For dose-response curves, separate groups of animals are injected with increasing doses of test drug and are tested at a predetermined time.

Data of some representative compounds using these measurements are summarized in Table 6.

**Table 6**

| **Anxiolytic Activity in Vogel Test in Rats** | | | | |
|---|---|---|---|---|
| **Compounds** | **Route** | **Vehicle** | **Dose (mg/kg)** | **Vogel (% of control)** |
| 3α,20α-Dihydroxy-2β-isopropoxy-5α-pregnane | i.c.v. | g-CD | 10 ug | 169.0 |
| 3α,20-Dihydroxy-20-methyl-5α-pregnane | i.c.v. | g-CD | 20 ug | 179.0 |
| 3α,20α-Dihydroxy-21-methyl-5α-pregnane | i.c.v. | g-CD | 10 ug | 157.9 |
| 3α,20α(S)-Dihydroxy-5α-pregnane | i.c.v. | b-CD | 10 ug | 193.0 |
| 3α-Hydroxy-5α-pregnan-20-one | i.c.v. | b-CD | 10 ug | 431.1 |
| 3α,20α-Dihydroxy-5β-pregnane | i.c.v. | g-CD | 10 ug | 283.3 |
| 2β-Fluoro-3α,20α-dihydroxy-5α-pregnane | i.c.v. | g-CD | 20 ug | 264.9 |
| 3α,20α-Dihydroxy-21-ethyl-5α-pregnane | i.c.v. | g-CD | 20 ug | 225.8 |
| 3α,20β-Dihydroxy-5β-pregnane | i.c.v. | g-CD | 20 ug | 267.3 |

### Prodrugs

Anti-convulsant and anxiolytic activities of prodrugs of the basic compounds 3α-hydroxy-5α-pregnan-20-one and 3α,21-dihydroxy-5α-pregnan-20-one and their derivatives were assessed as using the same procedures described above. Percent protection by several prodrugs of 3α-hydroxy-5α-pregnan-20-one against metrazol-induced seizures was plotted against time after administration of the compounds. (Table 7).

Several additional prodrugs were also tested and results are shown in Table 8. Modification of the basic compounds 3α-hydroxy-5α-pregnan-20-one and 3α,21-dihydroxy-5α-pregnan-20-one at the 3α and 21 hydroxyls with various esters maintains their biological activity and in some cases such modification increased the time of protection provided by the compound. Thus, the compounds of this invention can be modified to provide anti-convulsant and anxiolytic activities over a period of time, with varying degrees of protection.

**Table 7.**

| **Anti-Metrazol Activity of Prodrug Esters of 3α- Hydroxy-5α-pregnane-20-one (3α-(RCOO)-5α- pregnan-20-one)** | |
|---|---|
| **R** | **% Protection 60 mg/kg 1 hr, IP** |
| Methyl | 25 |
| Ethyl | 75 |
| Propyl | 75 |
| Butyl | 33 |
| 2-Propyl | 75 |
| 4-Heptyl | 16 (4 hour) |
| Cyclobutyl | 17 |
| Phenyl | 33 |
| 4-Chlorophenyl | 50 |
| 4-Methoxyphenyl | 17 |
| 3-Pyridyl | 50 (20 hour) |
| 3-(1-Methyl-1,4-dihydropyridinyl) | 63 (20 hour) |

In contrast to benzodiazepines, neuroactive steroids can also induce anesthesia. Their ability to induce anesthesia is thought to be due to their ability to open the chloride ion channel in the absence of GABA, which is a property not possessed by benzodiazepines. Therefore, neurosteroids can act directly in the absence of GABA, at the receptor, and also "indirectly", in the presence of GABA. This "indirect" action is called "modulating" the receptor. Lambert, *et al., Trends Pharmacology Science 8*: 224-227 (1987).

The compounds of and used in the invention can also be used for anesthetic indications at high doses. However, the preferred route of administration to induce anesthesia is intravenous (i.v.) administration. In animals, a drug's anesthetic properties is measured by the drug's ability to produce a loss-of-righting reflex. The loss-of-righting reflex is defined as the inability of an animal to right itself within 30 seconds when placed on its back. Mice were administered drug i.v. in the lateral tail vein. Following administration, mice were placed on their backs and observed for loss-of-righting reflex. Illustrative results are presented in Table 8.

**Table 8.**

| **Anesthetic Activity of Neuroactive Steroids in Mice** | | | | |
|---|---|---|---|---|
| **Compounds** | **Route** | **Vehicle** | **Dose (mg/kg)** | **Loss-of- Righting Reflex** |
| 3α,21-Dihydroxy-3β-ethynyl-5β-pregnan-20-one | iv | 20% cremophor | 10 | 100 |
| 3β-(Chloroethynyl)-3α-hydroxy-5β-prognan-20-one | iv | micronizing solution | 20 | 100 |
| 3β-Ethynyl-3α-hydroxy-5β-pregnan-20-one | iv | 10% hpbcd | 30 | 100 |
| 3α-Hydroxy-3β-methyl-5α-pregn-16-en-20-one | iv | micronizing solution | 50 | 100 |
| 3α-Hydroxy-5α-pregn-9-en-20-one | iv | micronizing solution | 10 | 100 |
| 3α-Hydroxy-17(20)(Z)-methoxytnethylene-19-nor-5α-androstane | iv | micronizing solution | 5 | 75 |
| 3α-Hydroxy-3β-methyl-5β-pregnan-20-one | iv | micronizing solution | 2.5 | 100 |
| 2β-Ethoxy-3α-hydroxy-5α-pregnan-20-one | iv | 20% cremophor | 5 | 100 |
| 2β-Fluoro-3α-hydroxy-5α-pregnan-20-one | iv | micronizing solution | 5 | 100 |
| 3α-Hydroxy-3β-methyl-21-methoxymethyl-5α-pregnan-20-one | iv | micronizing solution | 20 | 100 |

## Claims

1. A compound selected from the group consisting of:
3α-hydroxy-3β-(3'-methyl-but-3'-en-1'-ynyl)-5β-pregnan-20-one;
3α-hydroxy-3β-(3'-methyl-but-3'-en-1'-ynyl)-5α-pregnan-20-one;
3β-(cyclopropyl)ethynyl-3α-hydroxy-5β-pregnan-20-one; and
3α,21-dihydroxy-3β-fluoromethyl-5α-pregnan-20-one; or
a physiologically acceptable 3-ester, 20-ester, 21-ester, 3,20-diester, or 3,21-diester thereof.

2. A compound selected from the group consisting of:
3α,21-dihydroxy-3β-trifluoromethyl-19-nor-5β-pregnan-20-one, 21 -hemisuccinate, sodium salt;
3α,20α-dihydroxy-21-methyl-5α-pregnane, bis hemisuccinate;
3α,21-dihydroxy-3β-ethenyl-5α-pregnan-20-one, 21-αhemisuccinate;
3α,21-dihydroxy-3β-trifluoromethyl-5β-pregnan-20-one, 21 hemifumarate sodium salt;
3α,21-dihydroxy-3β-trifluoromethyl-5β-pregnan-20-one, methyl 21-succinate;
3α,21-dihydroxy-3β-trifluoromethyl-5β-pregnan-20-one, 21-propionate;
bis (3α,21-dihydroxy-3β-trifluoromethyl-5β-pregnan-20-one) 21-hemisuccinate; and
bis (3α,21-dihydroxy-3β-ethynyl-5β-pregnan-20-one) 21-hemisuccinate.

3. A compound as claimed in claim 1, which is 3α-hydroxy-3β-(3'methyl-but-3'-en-1'-ynyl)-5β-pregnan-20-one.

4. A compound as claimed in claim 2, which is 3α,21-dihydroxy-3β-trifluoromethyl-19-nor-5β-pregnan-20-one, 21-hemisuccinate, sodium salt.

5. A compound as claimed in claim 1, which is 3β-(cyclopropyl)ethynyl-3α-hydroxy-5β-pregnan-20-one.

6. A pharmaceutical composition, comprising an effective amount of a compound of any one of claims 1 to 5; and a pharmaceutically acceptable carrier or diluent.

7. The use of a compound as claimed in any one of claims 1 to 5 in the manufacture of a medicament for the treatment of stress or anxiety in an animal subject.

8. The use of a compound as claimed in any one of claims 1 to 5 in the manufacture of a medicament for alleviating seizure activity in an animal subject.

9. The use of a compound as claimed in any one of claims 1 to 5 in the manufacture of a medicament for reducing or alleviating insomnia in an animal subject.

10. The use of a compound as claimed in any one of claims 1 to 5 in the manufacture of a medicament for the treatment of mood disorders in an animal subject.

11. The use as claimed in claim 10 wherein said mood disorder is depression

12. The use of a compound as claimed in any one of claims 1 to 5 in the manufacture of a medicament for the treatment of pre-menstrual syndrome or post-natal depression in an animal subject.

13. The use of a compound as claimed in any one of claims 1 to 5 in the manufacture of a medicament for inducing anaesthesia in an animal subject.

## Patentansprüche

1. Verbindung ausgewählt aus der Gruppe bestehend aus:
3α-hydroxy-3β-(3'-methyl-but-3'-en-1'-ynyl)-5β-pregnan-20-on;
3α-hydroxy-3β-(3'-β-methyl-but-3'-en-1'-ynyl)-5α-pregnan-20-on;
3β-(cyclopropyl)ethynyl-3α-hydroxy-5β-pregnan-20-on; und
3α,21-dihydroxy-3β-fluoromethyl-5α-pregnan-20-on; oder
ein physiologisch annehmbarer 3-Ester, 20-Ester, 21-Ester 3,20-Diester, oder 3,21-Diester davon.

2. Verbindung ausgewählt aus der Gruppe bestehend aus:
3α,21-dihydroxy-3β-trifluoromethyl-19-nor-5β-pregnan-20-on,21-Hemisuccinat, Natriumsalz;
3α,20α-dihydroxy-21-methyl-5α-pregnan, bis Hemisuccinat;
3α,21-dihydroxy-3β-ethenyl-5α-pregnan-20-on, 21 -Hemisuccinat;
3α,21-dihydroxy-3β-trifluoromethyl-5β-pregnan-20-on,21 Hemifumarat Natriumsalz;
3α,21-dihydroxy-3β-trifluoromethyl-5β-pregnan-20-on, Methyl 21 -Succinat;
3α,21-dihydroxy-3β-trifluoromethyl-5β-pregnan-20-on, 21-Propionat;
bis (3α,21-dihydroxy-3β-trifluoromethyl-5β-pregnan-20-on) 21-Hemisuccinat; und
bis (3α,21-dihydroxy-3β-ethynyl-5β-pregnan-20-on) 21-Hemisuccinat.

3. Verbindung nach Anspruch 1, welche 3α-hydroxy-3β-(3'-methyl-but-3'-en-1'-ynyl)-5β-pregnan-20-on ist.

4. Verbindung nach Anspruch 2, welche 3α,21-dihydroxy-3β-trifluoromethyl-19-nor-5β-pregnan-20-on,21-Hemisuccinat, Natriumsalz ist.

5. Verbindung nach Anspruch 1, welche 3β-(cyclopropyl)ethynyl-3α-hydroxy-5β-pregnan-20-on ist.

6. Pharmazeutische Zusammensetzung, welche eine wirksame Menge einer Verbindung gemäß einem der Ansprüche 1 bis 5 und einen pharmazeutisch annehmbaren Träger oder ein solches Verdünnungsmittel enthält.

7. Verwendung einer Verbindung gemäß einem der Ansprüche 1 bis 5 bei der Herstellung eines Medikaments zur Behandlung von Stress oder Angstzuständen eines tierischen Wesens.

8. Verwendung einer Verbindung gemäß einem der Ansprüche 1 bis 5, bei der Herstellung eines Medikaments zur Milderung von Anfallsgeschehen eines tierischen Wesens.

9. Verwendung einer Verbindung gemäß einem der Ansprüche 1 bis 5 bei der Herstellung eines Medikaments zur Verminderung oder Milderung von Schlaflosigkeit eines tierischen Wesens.

10. Verwendung einer Verbindung gemäß einem der Ansprüche 1 bis 5 bei der Herstellung eines Medikaments zur Behandlung von Gemütsstörungen eines tierischen Wesens.

11. Verwendung nach Anspruch 10, wobei die genannte Gemütsstörung Depression ist.

12. Verwendung einer Verbindung gemäß einem der Ansprüche 1 bis 5 bei der Herstellung eines Medikaments zur Behandlung von prämenstrualem Syndrom oder postnataler Depression eines tierischen Wesens.

13. Verwendung einer Verbindung gemäß einem der Ansprüche 1 bis 5 bei der Herstellung eines Medikaments zur Auslösung von Betäubung eines tierischen Wesens.

## Revendications

1. Composé choisi parmi le groupe constitué de :
la 3α-hydroxy-3β-(3'-méthylbut-3'-én-1'-ynyl)-5β-prégnan-20-one ;
la 3α-hydroxy-3β-(3'-méthylbut-3'-én-1'-ynyl)-5α-prégnan-20-one ;
la 3β-(cyclopropyl)éthynyl-3α-hydroxy-5β-prégnan-20-one ; et
la 3α,21-dihydroxy-3β-fluorométhyl-5α-prégnan-20-one ; ou
un 3-ester, un 20-ester, un 21-ester, un 3,20-diester, ou un 3,21-diester physiologiquement acceptable de celles-ci.

2. Composé choisi parmi le groupe constitué de :
le sel sodique du 21-hémisuccinate de 3α,21-dihydroxy-3β-trifluorométhyl-19-nor-5β-prégnan-20-one ;
le bis-hémisuccinate de 3α,20α-dihydroxy-21-méthyl-5α-prégnane ;
le 21-hémisuccinate de 3α,21-dihydroxy-3β-éthényl-5α-prégnan-20-one ;
le sel sodique du 21-hémifumarate de 3α,21-dihydroxy-3β-trifluorométhyl-5β-prégnan-20-one ;
le 21-succinate de méthyle de la 3α,21-dihydroxy-3β-trifluorométhyl-5β-prégnan-20-one ;
le 21-propionate de 3α,21-dihydroxy-3β-trifluorométhyl-5β-prégnan-20-one ;
le 21-hémisuccinate de la bis(3α,21-dihydroxy-3β-trifluorométhyl-5β-prégnan-20-one) ; et
le 21-hémisuccinate de bis(3α,21-dihydroxy-3β-éthynyl-5β-prégnan-20-one).

3. Composé selon la revendication 1, qui est la 3α-hydroxy-3β-(3'-méthylbut-3'-én-1'-ynyl)-5β-prégnan-20-one.

4. Composé selon la revendication 2, qui est le sel sodique du 21-hémisucicinate de la 3α,21-dihydroxy-3β-trifluorométhyl-19-nor-5β-prégnan-20-one.

5. Composé selon la revendication 1, qui est la 3β-(cyclopropyl)éthynyl-3α-hydroxy-5β-prégnan-20-one.

6. Composition pharmaceutique comprenant une quantité efficace d'un composé selon l'une quelconque des revendications 1 à 5, et un support ou diluant pharmaceutiquement acceptable.

7. Utilisation d'un composé selon l'une quelconque des revendications 1 à 5 dans la fabrication d'un médicament pour le traitement du stress ou de l'anxiété chez un sujet animal.

8. Utilisation d'un composé selon l'une quelconque des revendications 1 à 5 dans la fabrication d'un médicament pour soulager l'activité de crise chez un sujet animal.

9. Utilisation d'un composé selon l'une quelconque des revendications 1 à 5 dans la fabrication d'un médicament pour réduire ou soulager l'insomnie chez un sujet animal.

10. Utilisation d'un composé selon l'une quelconque des revendications 1 à 5 dans la fabrication d'un médicament pour le traitement des maladies de l'humeur chez un sujet animal.

11. Utilisation selon la revendication 10 dans laquelle la maladie de l'humeur est la dépression.

12. Utilisation d'un composé selon l'une quelconque des revendications 1 à 5 dans la fabrication d'un médicament pour le traitement du syndrome prémenstruel ou de la dépression post-natale chez un sujet animal.

13. Utilisation d'un composé selon l'une quelconque des revendications 1 à 5 dans la fabrication d'un médicament pour induire l'anesthésie chez un sujet animal.
